# EUROPEAN PATENT SPECIFICATION

(11) **EP 3 395 325 B1**
(45) Date of publication and mention of the grant of the patent: **05.06.2024**
(21) Application number: 16878759.6
(22) Date of filing: 21.12.2016
(51) Int. Cl.: A61K 8/06, A45D 34/04, A61K 8/04, A61K 8/19, A61K 8/81, A61Q 1/10, A61K 8/73

(54) **LIQUID COSMETIC COMPOSITION**
FLÜSSIGE KOSMETISCHE ZUSAMMENSETZUNG
COMPOSITION COSMÉTIQUE LIQUIDE

(30) Priority: 25.12.2015 JP 2015253516; 25.12.2015 JP 2015253517
(43) Date of publication of application: 31.10.2018
(73) Proprietor: Mitsubishi Pencil Company, Limited, Tokyo 140-8537 (JP)
(72) Inventor: SAKUMA Satoshi, Fujioka-shi Gunma 375-8501 (JP); SATOU Hiroshi, Fujioka-shi Gunma 375-8501 (JP)
(74) Representative: Vossius & Partner Patentanwälte Rechtsanwälte mbB
(86) International application number: PCT/JP2016/088089
(87) International publication number: WO 2017/110872

(56) References cited:
- EP-A1- 2 353 583
- WO-A1-2007/083753
- WO-A1-2012/160649
- JP-A- 2003 073 220
- JP-A- 2005 052 649
- JP-A- 2008 030 335
- JP-A- 2008 055 742
- JP-A- 2008 063 283
- JP-A- 2009 096 769
- JP-A- 2009 292 772
- JP-A- 2014 118 498
- JP-A- 2014 169 266
- JP-A- 2015 209 394
- JP-U- 3 176 100
- "Color Nuanced Mascara Gel", GNPD,, 1 February 2010 (2010-02-01), XP002687044,

## Description

### Technical Field

The present invention relates to a liquid cosmetic composition suitable for an eyeliner, an eye shadow, an eyebrow and the like appropriate for point makeup around eyes, and more specifically, it relates to a liquid cosmetic composition suitable for eye makeup, which does not get blurred on the skin when applied thereon and drops (drips) less and which has a low viscosity and can drawn lines densely and is improved in a durability in makeup.

### Background Art

Cosmetics of a nonaqueous type and an emulsion type have so far been known as a cosmetic for point makeup around eyes. In recent years, liquid cosmetic compositions of an aqueous type are increasing in terms of spreading in applying and a makeup durability.

Known as the liquid cosmetic compositions of an aqueous type are, for example, (1) aqueous point makeup cosmetics characterized by containing an aqueous thickening gelling agent and pearl lustrous powder (refer to, for example, a patent document 1), (2) liquid cosmetics comprising at least an inorganic pigment, a dispersant, a film-forming agent and water, wherein the above dispersant is a homopolymer or a copolymer prepared by using as raw material monomers, one or more compounds selected from acrylic acid, methacrylic acid or (C₁ to C₄ and C₈)alkyl esters thereof; and the above film-forming agent is an emulsion of a homopolymer or a copolymer prepared by using as raw material monomers, one or more compounds selected from acrylic acid, methacrylic acid or (C₁ to C₄ and C₈)alkyl esters thereof, or styrene (refer to, for example, a patent document 2), (3) cosmetic compositions comprising a pigment composition containing an inorganic pigment or an inorganic filler and phosphoric ester having a specific structure (refer to, for example, a patent document 3), (4) oil-in-water type emulsion cosmetics comprising 15 to 35 % by mass of an acrylic acid base polymer prepared by polymerizing one or more monomers selected from the group consisting of acrylic acid, methacrylic acid, acrylic esters and methacrylic esters, 5 to 30 % by mass of a solid oil, 2 to 20 % by mass of a liquid oil, 0.2 to 5 % by mass of 12-hydroxystearic acid, 0.5 to 4 % by mass of solid fatty acid other than 12-hydroxystearic acid, 1 to 10 % by mass of a surfactant, and water (refer to, for example, a patent document 4), (5) oil-in-water type emulsion hair cosmetics comprising 5 to 15 % by mass of an alkylene oxide derivative represented by a specific formula, 0.5 to 3 % by mass of a polyoxyethylene adduct represented by a specific formula, 0.1 to 1 % by mass of sesquisostearic acid sorbitan, 5 to 30 % by mass of a wax, and water (refer to, for example, a patent document 5), and (6) cosmetics for eyelashes comprising a hydrophobic porous powder (silica) and a skin-forming agent (refer to, for example, a patent document 6).

On the other hand, known as cosmetics and the like prepared by using fermented celluloses are, for example, cosmetics characterized by controlling a stickiness by adding fermented cellulose to cosmetics in order to provide a cosmetic which controls a stickiness in applying cosmetics such as creams, gels and the like on the skin to provide the cosmetics with a good use feeling (refer to, for example, a patent document 7), and dispersion improving agents characterized by enhancing a dispersibility of a fermented cellulose dispersion, comprising hydroxyethyl cellulose and/or guar gum (refer to, for example, a patent document 8).

However, when the liquid cosmetic compositions of an aqueous type shown in the patent documents 1 to 6 are used for an applicator having a coating part using a brush comprising ultra fine fiber bundles which are suitable for point makeup around eyes, involved therein is the problem that because the whole brush is fine, a retaining amount of the liquid is very small and that the drawn lines become blurred and are roughened. Also, when the liquid is reduced in a viscosity in order to increase the retaining amount, the problem that the liquid drops (drips) is brought about. Further, when pigments having a conventional average particle diameter are used, the existing state is that involved therein is the problem that the pigments having a high specific gravity settle down to cause a difference in a hue between an upper part and a lower part in a long and narrow brush and that uneven colors are produced on the drawn lines.

Also, the cosmetics and the like prepared by using the fermented celluloses described in the patent documents 7 and 8 have the subject and the object of controlling a stickiness in applying cosmetics such as creams, gels and the like on the skin, and they use hydroxyethyl cellulose and the like in combination in order to enhance a dispersibility of a fermented cellulose dispersion. Thus, they are different in a subject, an object and a technical concept (a constitution and an action effect) from the liquid cosmetic composition of the present invention which does not become blurred on the skin when applied thereon to produce no uneven colors and which has a low viscosity to cause less dropping (dripping) and makes it possible to draw densely lustrous lines.

### Prior Art Documents

### Patent documents

Patent document 1: JP-A 2005-97148 (claims, examples and the like)
Patent document 2: International Publication WO2007/083753 (claims, examples and the like)
Patent document 3: JP-A (through PCT) 2014-508770 (claims, examples and the like)
Patent document 4: JP-A 2010-138110 (claims, examples and the like)
Patent document 5: JP-A 2010-280632 (claims, examples and the like)
Patent document 6: JP-A 2006-111536 (claims, examples and the like)
Patent document 7: JP-A 2009-96769 (claims, examples and the like)
Patent document 8: JP-A 2015-93898 (claims, examples and the like)

### Disclosure of the Invention

### < Problems to be solved by the Invention >

In light of the problems and the existing state each described above, the present invention intends to solve them, and an object thereof is to provide a liquid cosmetic composition suitable for eye makeup, wherein it does not get blurred on the skin when applied thereon even in a case where it is used for an applicator having a coating part using a brush comprising ultra fine fiber bundles which are suitable for point makeup around eyes; it drops (drips) less; it has a low viscosity and makes it possible to densely draw lines; and it is improved in a durability in makeup.

### < Means to solve the Problems >

In light of the conventional problems described above and the like, intense researches repeated by the present inventors have resulted in finding that the liquid cosmetic composition which meets the object described above is obtained by adding an aqueous emulsion of an acrylic acid base polymer having specific physical properties obtained by polymerizing monomers selected from an acrylic acid monomer, a methacrylic acid monomer, an acrylic ester monomer and a methacrylic ester monomer, cellulose having specific physical properties, a specific color material, and water. Thus, they have come to complete the present invention.

That is, the present invention resides in the following items (1) to (11).
(1) A liquid cosmetic composition comprising at least an aqueous emulsion of an acrylic acid base polymer having a glass transition point of 20°C or lower obtained by polymerizing monomers selected from an acrylic acid monomer, a methacrylic acid monomer, an acrylic ester monomer and a methacrylic ester monomer, crystalline cellulose, an inorganic pigment, and water.
(2) The liquid cosmetic composition as described in the above item (1), comprising 2 to 30 % by mass (in terms of a solid content) of the aqueous emulsion of the acrylic acid base polymer, 0.1 to 3.0 % by mass of the crystalline cellulose and 1 to 30 % by mass of the inorganic pigment.
(3) The liquid cosmetic composition as described in the above item (1) or (2), wherein iron oxide particles are contained as the inorganic pigment.
(4) The liquid cosmetic composition as described in any one of the above items (1) to (3), wherein a viscosity at a temperature of 25°C and a shear rate of 3.83 s⁻¹ is 20 to 500 mPa·s.
(5) A liquid cosmetic composition comprising at least an aqueous emulsion of an acrylic acid base polymer having a glass transition point of 20°C or lower obtained by polymerizing monomers selected from an acrylic acid monomer, a methacrylic acid monomer, an acrylic ester monomer and a methacrylic ester monomer, fermented cellulose, a color material containing at least a tabular pigment, and water.
(6) The liquid cosmetic composition as described in the above item (5), comprising 2 to 20 % by mass (in terms of a solid content) of the aqueous emulsion of the acrylic acid base polymer, 0.05 to 0.6 % by mass of the fermented cellulose and 1 to 30 % by mass of the color material containing at least the tabular pigment.
(7) The liquid cosmetic composition as described in the above item (5) or (6), wherein iron oxide particles are contained as the color material, and a chelating agent is further contained.
(8) The liquid cosmetic composition as described in any one of the above items (5) to (7), wherein a viscosity at a temperature of 25°C and a shear rate of 76.6 s⁻¹ is 30 to 200 mPa·s.
(9) The liquid cosmetic composition as described in any one of the above items (1) to (8), wherein it is a liquid cosmetic composition for eye makeup.
(10) A liquid cosmetic applicator equipped with at least a coating part and a coating liquid reservoir, wherein the coating part comprises a brush; fibers of the brush include those having a cross section other than a circle; and the liquid cosmetic composition as described in any one of the above items (1) to (9) is stored in the coating liquid reservoir.
(11) The liquid cosmetic applicator as described in the above item (10), wherein all the fibers of the brush have cross sections other than a circle.

### < Effects of the Invention >

According to the present invention, provided is a liquid cosmetic composition which does not become blurred on the skin, does not produce uneven colors and drops (drips) less and which has a low viscosity and draws lines densely. Also, provided is a liquid cosmetic composition which is prevented from producing uneven colors due to a difference in a hue between an upper part and a lower part even when a long and narrow brush is used and which draws lines densely and is improved in a durability in makeup.

### BRIEF DESCRIPTION of THE DRAWINGS

FIG. 1 shows one example of the embodiment of the liquid cosmetic applicator of the present invention and is a vertical cross section showing the situation before use.
FIG. 2 shows one example of the embodiment of the liquid cosmetic applicator of the present invention and is a vertical cross section showing a use starting situation.
FIG. 3 is an enlarged side view showing one example of the brush shown in FIG. 1.
FIG. 4 is a side view showing one example of the brush hair shown in FIG. 3.
FIG. 5 is a side view showing another example of the brush hair shown in FIG. 3.
FIG. 6 is a side view showing a modified example of the brush hair shown in FIG. 5.
FIG. 7 is a perspective view showing a modified example in which a concave part is formed in the brush hair shown in FIG. 4.
FIG. 8 is a perspective view showing a modified example in which a concave part is formed in the brush hair shown in FIG. 5.
FIG. 9 is a partial vertical cross section showing another example of the embodiment of the liquid cosmetic applicator of the present invention.
FIG. 10 is a partial vertical cross section showing another example of the embodiment of the liquid cosmetic applicator of the present invention.

### MODE FOR CARRYING OUT THE INVENTION

The embodiments of the present invention shall be explained below in detail.

The liquid cosmetic composition of the present invention is characterized in the first invention by comprising at least an aqueous emulsion of an acrylic acid base polymer having a glass transition point of 20°C or lower obtained by polymerizing monomers selected from an acrylic acid monomer, a methacrylic acid monomer, an acrylic ester monomer and a methacrylic ester monomer, crystalline cellulose, an inorganic pigment, and water. Also, the liquid cosmetic composition of the present invention is characterized in the second invention by comprising at least an aqueous emulsion of an acrylic acid base polymer having a glass transition point of 20°C or lower obtained by polymerizing monomers selected from an acrylic acid monomer, a methacrylic acid monomer, an acrylic ester monomer and a methacrylic ester monomer, fermented cellulose, a color material containing at least a tabular pigment, and water.

The first invention and the second invention shall be explained below in order. Also, the term referred to as "the present invention" includes the first invention and the second invention.

### Liquid cosmetic composition of the first invention:

The aqueous emulsion of the acrylic acid base polymer shall not specifically be restricted as long as it is an aqueous emulsion of an acrylic acid base polymer having a glass transition point of 20°C or lower obtained by polymerizing one or more kinds of monomers selected from an acrylic acid monomer, a methacrylic acid monomer, an acrylic ester monomer and a methacrylic ester monomer, and various aqueous emulsions can be used.

The aqueous emulsion of the acrylic acid base polymer is used as a coating film forming agent, and an aqueous emulsion having a glass transition point of -20°C or higher and 20°C or lower is preferably used from the viewpoints of a film forming property and an elasticity of the coating film formed. The aqueous emulsion having a glass transition point exceeding 20°C is reduced in an elasticity and a coating film forming property of a dried cosmetic and reduced in a durability, and therefore it is not preferred.

In the present invention, the glass transition point (Tg) was measured in the following manner. First, the resin was turned into a lacquer, and a film was formed from the resin turned into a lacquer. Then, the solvent was removed by drying the film to obtain a dried film. The acryl base resin turned into the film was heated from -20°C to +150°C at a heating rate of 10°C/minute by means of a differential scanning calorimeter (DSC) manufactured by Rigaku Corporation to measure a differential scanning calorie. The endothermic curve obtained was differentiated to determine an inflection point, and this inflection point was judged as Tg.

Yodosol GH800 (glass transition point: -15°C, solid content: 45 %, manufactured by Akzo Nobel Corporation), Yodosol GH810F (glass transition point: 10°C, solid content: 46 %, manufactured by Akzo Nobel Corporation) which are alkyl acrylate copolymer emulsions, COVACRYL MS11 (glass transition point: 0°C, solid content: 50 %, manufactured by Sensient Technologies Corporation) which is an alkyl acrylate copolymer emulsion, Daitosol 5000AD (glass transition point: -14°C, solid content: 50 %, manufactured by Daito Kasei Kogyo Co., Ltd.) which is an alkyl acrylate copolymer emulsion, and the like can be used as the aqueous emulsion of the acrylic acid base polymer which can specifically be used.

For example, 2-amino-2-methyl-1-propanol, 2-amino-2-methyl-1,3-propanediol, triethanolamine, L-arginine, aqueous ammonia, sodium hydroxide, and the like may be used, if necessary, for neutralization.

A content of the aqueous emulsion of the acrylic acid base polymer is preferably 2 to 30 % by mass (hereinafter, % by mass is referred to merely as "ε"), further preferably 2 to 20 % and more preferably 5 to 15 % on the whole amount of the liquid cosmetic composition in terms of a solid content (resin content).

The water resistance can be enhanced by controlling the content of the aqueous emulsion to 2 % or more in terms of a solid content (resin content). On the other hand, the good coating performance can be exerted without drying the coating part of the applicator by controlling the content of the aqueous emulsion to 30 % or less in terms of a solid content (resin content).

In the aqueous emulsion which is a coating film forming agent, a surfactant is used in a certain case for the purpose of stabilizing the emulsion itself. In this regard, an addition amount of the surfactant is not specifically restricted since it exerts less influence on the water resistance and the skin adhesiveness which are the performances of the liquid cosmetic.

The crystalline cellulose used in the present first invention functions not only as a thickener but also as a settling preventive which prevents the pigments from setting down into a hard solid and a cushioning material which gets into the pigments. To be specific, it is a cellulose crystallite aggregate having a substantially fixed polymerization degree which is obtained by subjecting cellulose to acid hydrolysis or alkali oxidative decomposition, and it has a characteristic in which cellulose crystals are turned into dispersed particles in water unlike an aqueous solution of conventional thickening polysaccharides such as a xanthan gum and the like.

The crystalline cellulose which can be used includes, for example, celluloses comprising colloidal grouds obtained by subjecting the surfaces of fine cellulose crystals of primary particles to coating treatment with a water-soluble polymer and the like, and it includes, for example, Ceolus RC-591, RC-N81, RC-591NF, CL-611 and Ceolus Cream (all manufactured by Asahi Kasei Corporation, and the like.

A content of the crystalline cellulose is preferably 0.1 to 3.0 %, more preferably 0.5 to 3.0 % based on the whole amount of the liquid cosmetic composition.

Controlling the content of the crystalline cellulose to 0.1 % or more makes it possible to enhance the viscosity, inhibit the cosmetic from flowing into the wrinkles on the skin by the cellulose particles and draw dense lines. On the other hand, controlling the content of the crystalline cellulose to 3.0 % or less makes it possible to draw lines extending well as is with a free ink type and a sliver type.

In the present first invention, the inorganic pigment is used as the color material.

The inorganic pigment used in the present first invention shall not specifically be restricted, and those usually used as a pigment of an aqueous cosmetic can be used. It includes, for example, at least one (alone or a mixture of two or more kinds thereof respectively) selected from carbon blacks, iron oxide particles (black iron oxide particles, yellow iron oxide particles and the like), chromium oxide, ultramarine blue, Prussian blue, zinc oxide, aluminum oxide, silicon dioxide, titanium oxide, magnesium oxide, chromium hydroxide, calcium carbonate, titanium yellow, red iron oxide, pearl pigments (lustrous particles) which have a high specific gravity and are large particle size color materials, such as titanium dioxide-coated scaly titanium, red iron oxide-coated mica titanium, scale foils and Ns-lauroyl-L-lysine-coated talc.

Also, the inorganic pigments such as carbon blacks, iron oxide particles, ultramarine blue, Prussian blue, red iron oxide and the like may be subjected to processing treatment on the surfaces using a silicone-based surface treating agent from the viewpoint of enhancing more the dispersibility.

The respective colors having good hues are obtained by combining suitable pigments selected from the inorganic pigments described above. For example, the good hues of a black color, a brown color and the like can be displayed by selecting and using the inorganic pigments of a black color and a brown color such as carbon blacks, iron oxide particles, red iron oxide, Prussian blue and the like.

Also, from the viewpoints of exerting the good hues and enhancing the dispersibility, the inorganic pigments having an average particle diameter of preferably 300 nm (0.3 um) or less, more preferably 20 to 250 nm (0.02 to 0.25 µm) and particularly preferably 20 to 200 nm (0.02 to 0.2 µm) are desirably used.

Using the inorganic pigments having the average particle diameters described above makes it possible to realize the high dispersibility without causing aggregation in water, provide the satisfactory hue, prevent clogging in the applicator and exert the excellent discharge performance. In this regard, the average particle diameter referred to in the present invention is a value determined by measuring the diameters (25°C) of the pigment particles in the liquid cosmetic composition by means of a particle diameter measuring device FPAR-1000 (manufactured by Otsuka Electronics Co., Ltd.) according to a dynamic light scattering method.

A content of the inorganic pigments is preferably 1 to 30 %, more preferably 4 to 25 % based on the whole amount of the liquid cosmetic composition.

If the content thereof is less than 1 %, the color is developed lightly, and it is unsatisfactory for the cosmetic. On the other hand, if the content exceeds 30 %, the cosmetic composition is increased in a viscosity too much, and when it is used for a liquid cosmetic applicator, the liquid is not smoothly discharged, so that it is not preferred.

An organic pigment, a dye and the like other than the inorganic pigment described above used for the liquid cosmetic composition may be used as long as the effects of the present invention are not damaged.

In the liquid cosmetic composition of the present first invention, water (including refined water, distilled water, ion-exchanged water, purified water, ultra purified water and the like) is used as the solvent. A content of the water is a balance obtained by excluding the amounts of the respective components described above and optional components described later from the whole amount of the liquid cosmetic composition.

Further, in addition to the respective components described above, optional components used for conventional liquid cosmetic compositions can be added to the liquid cosmetic composition of the present invention. To be specific, surfactants, pigment dispersants, moisturizing agents, preservatives, antioxidants, neutralizing agents, UV absorbers, chelating agents, moisturizing agents, cosmetic ingredients, perfumes, viscosity controllers, and the like can suitably be added in suitable amounts as long as the effects of the present invention are not damaged.

Any kinds of surfactants of, for example, an anionic base, a cationic base and a nonionic base can be used as the usable surfactants. They include, to be specific, lecithin, propylene glycol fatty acid esters, glycerin fatty acid esters, polyglycerin fatty acid esters, polyoxyethylene alkyl ethers, polyoxyethylene polyoxypropylene alkyl ethers, polyoxyethylene alkyl ether phosphoric acid (phosphoric esters), polyethylene glycol fatty acid esters, alkylsulfates (sulfonic esters), polyoxyethylene alkyl ether sulfates, and the like.

A content of the surfactants described above is preferably 0.5 %, or less based on the whole amount of the liquid cosmetic. If the content exceeds 0.5 %, the liquid cosmetic is inferior in a water resistance and cannot provide a sufficiently high fixing power.

In the present first invention, a dispersant can be added, if necessary, in order to further enhance a dispersibility of the pigment.

The dispersant which can be used shall not specifically be restricted, and AMPHOMER HC, Aquadure SPA-30 (sodium polyaspartate, manufactured by'Ajinomoto Co., Inc.), NIKKO BB30 (behenyl polyoxyethylene 30 mole adduct, manufactured by Nikko Chemicals Co., Ltd.), and the like can be used. The above dispersants can be added in an amount of 0.1 to 5 % based on the whole amount of the liquid cosmetic.

The chelating agents which can be used include, for example, edetic acid or salts thereof, ethylenediaminetriacetic acid or salts thereof, gluconic acid or salts thereof, phytic acid or salts thereof, pyrophosphoric acid or salts thereof, polyphosphoric acid or salts thereof, metaphosphoric acid or salts thereof, hydroxyethanediphosphonic acid or salts thereof, citric acid or salts thereof, tartaric acid, gluconic acid, phytic acid, and the like. The above chelating agents can be added in an amount of 0.01 to 0.5 % based on the whole amount of the liquid cosmetic.

Capable of being used as the moisturizing agents in a suitable amount are, for example, glycols which are soluble in water, such as 1,3-butylene glycol, 1,4-butylene glycol, pentylene glycol, ethylene glycol, diethylene glycol, polyethylene glycol, propylene glycol, dipropylene glycol, glycerin, and the like.

The preservatives which can be used include parabens, sodium dehydroacetate, phenoxyethanol and the like. Fungicides are included in the preservatives used in the present invention, and methyl paraoxybenzoate, ethyl paraoxybenzoate, propyl paraoxybenzoate, butyl paraoxybenzoate, isopropyl paraoxybenzoate and the like can be used in a suitable amount as the parabens which are the preservatives.

The liquid cosmetic composition of the present first invention is controlled preferably to a viscosity of 20 to 500 mPa·s at a temperature of 25°C and a shear rate of 3.83 s⁻¹ (1 rpm) from the viewpoints of a stability and a coating property.

More preferable range in the viscosity range described above is varied depending on the kind of the blend components of the liquid cosmetic composition and the uses thereof (eyeliner, eye shadow, skin makeup and the like), and it is more preferably 20 to 300 mPa·s, particularly preferably 50 to 200 mPa·s.

Controlling the viscosity value to 20 mPa·s or more makes it possible to prevent dripping, inhibiting the cosmetic from flowing into the wrinkles and draw dense lines. On the other hand, controlling the viscosity value to 500 mPa·s or less makes it possible to discharge smoothly the liquid cosmetic stored in the liquid cosmetic applicator using a brush or a pen feed as an applying means according to the present invention.

In the present first invention, the viscosity measuring conditions (including examples and the like described later) mean, to be specific, values measured at the respective shear rates by means of a cone plate type viscometer (ELD type viscometer, manufactured by Toki Sangyo Co., Ltd.).

More preferably, the viscosity at a temperature of 25°C and a shear rate of 38.3 s⁻¹ (10 rpm) is preferably 10 to 70 mPa·s, and the viscosity at a shear rate of 383 s⁻¹ (100 rpm) is preferably 5 to 20 mPa·s.

Also, the viscosity can be controlled to the ranges described above by suitably combining the amounts of the respective components including the inorganic pigment such as carbon black and iron oxide particles, water, the aqueous emulsion which is the coating film forming agent, the crystalline cellulose and the like.

Further, a surface tension of the liquid cosmetic composition of the present first invention measured at a temperature of 25°C according to a Wilhelmy method (plate method) is controlled to the range of preferably 25 to 60 mN/m, more preferably 30 to 45 mN/m from the viewpoint of well spreading in applying. Feathering and unevenness are prevented from being produced on the drawn lines by controlling the surface tension to 25 mN/m or more to make it possible to apply smoothly the liquid cosmetic composition. Also, the coating liquid is improved in a followability and can smoothly be applied by controlling the surface tension to 60 mN/m or less.

In the present invention, the surface tension is a value measured at a temperature of 25°C by means of a CBVP-Z type surface tension meter (plate method) manufactured by Kyowa Interface Science Co., Ltd.

The surface tension can be controlled to the ranges described above by suitably combining the amounts of the respective components including the inorganic pigment such as carbon black and iron oxide particles, water, the aqueous emulsion which is the coating film forming agent, the crystalline cellulose and the like.

The liquid cosmetic composition of the present first invention constituted in the manner described above can be a liquid cosmetic which is decreased in settling and separating of the pigment in the inside of the storing part of the coating liquid before coating, that is, causes less color separation by comprising the aqueous emulsion of the acrylic acid base polymer having a glass transition point of 20°C or lower obtained by polymerizing monomers selected from an acrylic acid monomer, a methacrylic acid monomer, an acrylic ester monomer and a methacrylic ester monomer, the crystalline cellulose, the inorganic pigment, and water, and obtained is the liquid cosmetic composition which does not get blurred on the skin when applied thereon and drops (drips) less and which has a low viscosity and can draw lines densely.

Also, the liquid cosmetic composition of the present invention has the blend characteristic described above, and therefore obtained is the liquid cosmetic composition which is prevented from producing uneven colors in the drawn lines due to a difference in a hue between an upper part and a lower part even when a long and narrow brush is used and which can draw lines densely.

### Liquid cosmetic composition of the second invention:

The liquid cosmetic composition of the present second invention is characterized by comprising at least the aqueous emulsion of the acrylic acid base polymer having a glass transition point of 20°C or lower obtained by polymerizing monomers selected from an acrylic acid monomer, a methacrylic acid monomer, an acrylic ester monomer and a methacrylic ester monomer, the fermented cellulose, the color material containing at least a tabular pigment, and water.

The aqueous emulsion of the acrylic acid base polymer used in the present second invention is the same as the aqueous emulsion of the acrylic acid base polymer used in the present first invention described above, and therefore the explanations thereof (contents, addition amounts and the like) shall be omitted.

The fermented cellulose used in the present second invention is cellulose produced by cellulose producing bacteria classified into an acetobacter genus, a pseudomonas genus, an agrobactrium genus and the like, and it is obtained by culturing an acetic acid bacterium on a suitable culture medium under stirring while aerating to obtain bacterial cells and separating and recovering cellulose fibers from the bacterial cells obtained above. The resulting cellulose fibers are fibrous substances forming a very fine three-dimensional structure in an aqueous medium.

A fermented cellulose which can be used may be commercially available products, and may be those obtained by culturing acetobacters, such as Acetobacter xylinum, Acetobacter pasteurianus, etc. in culture media containing nutrients such as nitrogen source, carbon source, water and oxygen etc. For example, those prepared by fermenting flesh and juice of coconuts with cellulose producing bacteria such as the acetobacters above and other can be used for a fermented cellulose of the invention.

A content of the fermented cellulose is preferably 0.05 to 0.6 %, more preferably 0.07 to 0.4 % based on the whole amount of the liquid cosmetic composition.

Controlling the content of the fermented cellulose to 0.05 % or more makes it possible to inhibit the tabular pigment from settling down and draw lustrous and dense lines. On the hand, controlling the content to 0.6 % or less makes it possible to draw lustrous and well extending lines.

In the present second invention, the color material containing at least the tabular pigment is used as the color material.

The tabular pigment used in the present second invention includes, for example, at least one (alone or a mixture of two or more kinds thereof respectively; hereinafter the same shall apply) selected from mica titanium, carmine-coated mica titanium, chromium oxide-coated mica titanium, iron oxide-coated mica titanium, iron oxide·carmine-coated mica titanium, iron oxide·Prussian blue-coated mica titanium, ultramarine blue-coated mica titanium, Prussian blue-coated mica titanium, red iron oxide-coated mica, red iron oxide-coated mica titanium, red iron oxide·carmine-coated mica titanium, red iron oxide-iron oxide-coated mica titanium, red iron oxide·Prussian blue-coated mica titanium, red iron oxide-iron oxide·Prussian blue-coated mica titanium, and lustrous pigments prepared by coating metal or metal oxide on a glass flake or a massive flake used as a base material.

It includes preferably iron oxide-coated mica titanium, mica titanium and ultramarine blue-coated mica titanium from the viewpoint of an evenness and a coating property of the coating film and obtaining drawn lines having an excellent brightness, and iron oxide-coated mica titanium and mica titanium are particularly desirably used.

In the tabular pigments described above, the average particle diameter is preferably 5 to 100 µm, more preferably 10 to 60 um.

Blending the tabular pigments having the average particle diameter described above with the fermented cellulose makes it possible to prevent aggregation and realize the high dispersibility in water, provide the sufficiently high hue, inhibit the applicator from being clogged and materialize the excellent discharge performance. In this connection, the "average particle diameter" is a value determined by means of a digital microscope VHX-2000 (manufactured by Keyence Corporation) in the present second invention.

A content of the tabular pigments is 1 to 30 %, preferably 5 to 25 % based on the whole amount of the liquid cosmetic composition in order to obtain the coating performances, the use characteristics and the lustrous drawn lines.

Controlling the content of the tabular pigment to 1 % or more makes it possible to obtain the good use property and the lustrous and good coating property. On the hand, the content is controlled preferably to 30 % or less from the viewpoints of the coating performance, the use characteristics and the lustrous drawn lines.

In the present second invention, pigments other than the tabular pigment can be used from the viewpoint of coloring of the liquid cosmetic for eye makeup.

The pigment which can be used includes, for example, at least one of carbon blacks, iron oxide particles (black iron oxide particles, yellow iron oxide particles and the like), titanium oxide, Prussian blue, ultramarine blue, blue No. 1, red iron oxide, chromium oxide, chromium hydroxide, zinc oxide, zirconium oxide, cobalt oxide, scale foils, bismuth oxychloride, pigments such as blue No. 2, blue No. 404, red No. 201, red No. 202, red No. 220, red No. 102, red No. 104, yellow No. 4, and yellow No. 4 Al lake, and an aluminum-coating polyester film.

The suitable range of the content of the above pigments (excluding the tabular pigments described above) is adjusted within the range of the total content of the color materials including the above pigment and the tabular pigment.

A content of the color materials including the above tabular pigments is preferably 1 to 30 %, more preferably 5 to 25 % based on the whole amount of the liquid cosmetic composition.

If the content of the color materials including the above tabular pigments is 1 % or less, the color is developed lightly and is unsatisfactory for the cosmetic. On the other hand, if it exceeds 30 %, the viscosity is increased too high, the liquid is not smoothly discharged when the liquid cosmetic is used in the applicator, and it is not preferred.

Color materials such as organic pigments and dyes other than the pigments described above may be used as long as the effects of the present second invention are not damaged.

In the liquid cosmetic composition of the present second invention, water (including refined water, distilled water, ion-exchanged water, purified water, ultra purified water and the like) is used as the solvent. A content of the water is a balance obtained by excluding the amounts of the respective components described above and optional components described later from the whole amount of the liquid cosmetic composition.

Further, in addition to the respective components described above, optional components used for conventional liquid cosmetic compositions can be added to the liquid cosmetic composition of the present second invention. To be specific, surfactants, pigment dispersants, moisturizing agents, preservatives, antioxidants, neutralizing agents, UV absorbers, chelating agents, moisturizing agents, cosmetic ingredients, perfumes, viscosity controllers, and the like can be added in suitable amounts as long as the effects of the present invention are not damaged.

The surfactants which can be used, the dispersants which are added, if necessary, in order to further enhance a dispersibility of the pigments, the chelating agents, the moisturizing agents, the preservatives (including the fungicides), and the like are the same as the dispersants, the chelating agents, the moisturizing agents, the preservatives (including the fungicides) and the like which are used in the present first invention described above, and therefore the respective explanations thereof (the respective contents, the respective addition amounts and the like) shall be omitted.

The liquid cosmetic composition of the present second invention is controlled to a viscosity of preferably 30 to 200 mPa·s at a temperature of 25°C and a shear rate of 76.6 s⁻¹ (20 rpm) form the viewpoints of a stability and a coating property.

More preferable range in the viscosity range described above is varied depending on the kind of the blend components of the liquid cosmetic composition for eye makeup and the uses thereof (eyeliner, eye shadow, skin makeup and the like), and it is more preferably 40 to 150 mPa·s.

Controlling the viscosity value to 30 mPa·s or more makes it possible to draw lustrous and dense lines. On the other hand, controlling the viscosity value to 200 mPa·s or less makes it possible to cause the liquid cosmetic stored in a liquid cosmetic applicator using a brush or a pen feed as an applying means according to the present invention to be discharged smoothly.

In the present second invention, the viscosity measuring conditions (including examples and the like described later) mean, to be specific, values measured at the respective shear rates by means of the cone plate type viscometer (ELD type viscometer, manufactured by Toki Sangyo Co., Ltd.).

More preferably, the viscosity at a temperature of 25°C and a shear rate of 38.3 s⁻¹ (10 rpm) is 40 to 350 mPa·s, and the viscosity at a shear rate of 383 s⁻¹ (100 rpm) is desirably 20 to 70 mPa·s.

Also, the viscosity can be controlled to the ranges described above by suitably combining the amounts of the respective components such as the color material including the tabular pigment, water, the aqueous emulsion which is the coating film forming agent, the fermented cellulose and the like.

Further, a surface tension of the liquid cosmetic composition of the present second invention measured at a temperature of 25°C according to the Wilhelmy method (plate method) is, as is the case with the present first invention, controlled to the range of preferably 25 to 60 mN/m, more preferably 30 to 45 mN/m from the viewpoint of well extending in applying. Feathering and unevenness are not caused on the drawn lines to make it possible to apply smoothly the liquid cosmetic composition by controlling the surface tension to 25 mN/m or more. Also, the coating liquid is improved in a followability and can smoothly be applied by controlling the surface tension to 60 mN/m or less.

In the present second invention, the surface tension is, as is the case with the present first invention, a value measured at a temperature of 25°C by means of the CBVP-Z type surface tension meter (plate method) manufactured by Kyowa Interface Science Co., Ltd.

The surface tension can be controlled to the ranges described above by suitably combining the amounts of the respective components such as the color material including the tabular pigment, water, the aqueous emulsion which is the coating film forming agent, the fermented cellulose and the like.

In the liquid cosmetic composition of the present second invention constituted in the manner described above, the liquid cosmetic composition suited to eye makeup which inhibits settling and separating of the tabular pigment though a viscosity is low and can draw beautiful lines having an excellent brilliance with the color material containing the tabular pigment and which does not become blurred on the skin when applied thereon, does not cause unevenness of the brilliant colors, drops (drips) less, has a low viscosity and can draw densely lustrous lines is provided by comprising the aqueous emulsion of the acrylic acid base polymer having a glass transition point of 20°C or lower obtained by polymerizing monomers selected from an acrylic acid monomer, a methacrylic acid monomer, an acrylic ester monomer and a methacrylic ester monomer, the fermented cellulose, the color material containing at least the tabular pigment, and water.

Also, the liquid cosmetic composition of the present second invention has the blend characteristics described above, and therefore obtained is the liquid cosmetic composition which is prevented from producing uneven colors in the drawn lines due to a difference in a hue between an upper part and a lower part even when a long and narrow brush is used and which can draw lustrous lines densely.

### Liquid cosmetic applicator related to the first invention and the second invention:

The liquid cosmetic compositions of the first invention and the second invention which are constituted in the manners described above are stored in a liquid cosmetic applicator equipped with a brush or pen feed in a coating part and used.

In the present invention (first invention and second invention), a suitable liquid cosmetic applicator shall not specifically be restricted as long as it is provided with a structure in which a liquid cosmetic composition (coating liquid) can suitably be applied on the skin in the forms of an eyeliner, an eye shadow and an eyebrow each suited to point makeup around the eyes. It includes preferably liquid cosmetic applicators equipped with a sliver type container which is excellent in a usability, a simplicity and a coating property and which holds a liquid cosmetic in a sliver or a collector type container which holds a liquid cosmetic in a single sheet body, and liquid cosmetic applicators in which a liquid cosmetic composition is stored directly in a coating liquid storing part and in which a discharge mechanism of the coating liquid is knock type or a rotary (turning) type.

In the present invention (first invention and second invention), preferred is a liquid cosmetic applicator equipped with at least a coating part and a coating liquid storing part, wherein the coating part comprises a brush; the fibers of the brush have a cross-sectional shape other than a circular shape; and the liquid cosmetic composition of the present invention is stored in the coating liquid storing part described above.

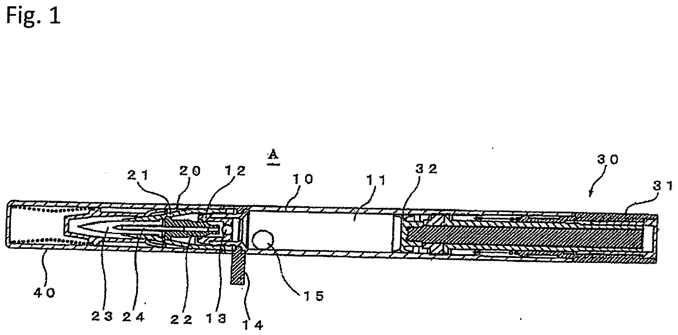

The liquid cosmetic applicator of the above form includes a liquid cosmetic applicator A shown in FIG. 1 to FIG. 8.

The liquid cosmetic applicator A of the this embodiment is, as shown in FIG. 1, equipped with a storing part (receiving part) 11 for a coating liquid which is the liquid cosmetic composition of the present invention having the constitution described above, which is provided in a front inside of a shaft body 10 as an applicator body, a stopper body 12 which is installed in a front end part of the a shaft body 10 and which closes tightly the storing part (receiving part) 11 before starting use, and a seal ball 13 which is installed in the stopper body 12 to maintain a tight closing state and which releases the tight closing state of the storing part (receiving part) by being detached from the stopper body 12 to allow the coating liquid to stay in a state of being discharged from the storing part (receiving part) 11.

Also, the liquid cosmetic applicator A is equipped with a stopper 14 which is detachably installed on an outer peripheral surface in the front end part of the shaft body 10 and a front shaft 20 which moves to a shaft direction by the length of the stopper 14 by detaching the stopper 14 in starting use and which is connected directly to the shaft body 10.

The above front shaft 20 is equipped with a front shaft body 21, a pipe joint 22 which is installed in the front shaft body 21 to make it possible to supply the coating liquid from the storing part 11 to a brush (coating part) 23 and a conduit tube 24 for connecting the pipe joint 22 with the brush (coating part) 23.

The liquid cosmetic applicator A is constituted in the manner described above, and therefore in a state (a state before starting use) shown in FIG. 1, the stopper 14 is installed in the shaft body 10, so that the storing part (receiving part) 11 is maintained in a tight closing state by the stopper body 12 (seal ball 13) closing tightly the storing part (receiving part) 11 without moving the front shaft 20.

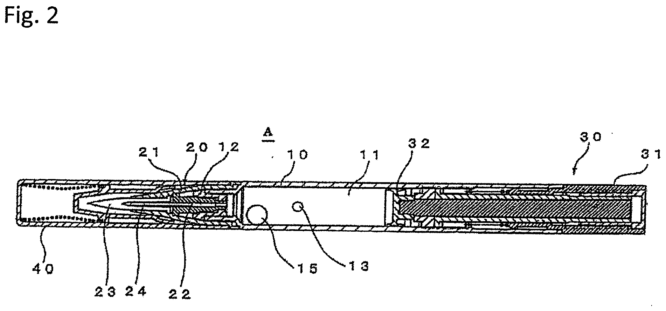

As shown in FIG. 2, the stopper 14 is detached in starting use, whereby the front shaft 20 moves to a shaft direction by the length of the stopper 14 and is brought into contact with the shaft body 10. That is, the pipe joint 22 of the front shaft 20 is connected with the stopper body 12, and the seal ball 13 installed in the stopper body 12 is detached by the front part of the pipe joint 22.

As the result thereof, the storing part (receiving part) 11 is released from the tight closing state, and the storing part (receiving part) 11 is allowed to be in a state in which the coating liquid can be supplied from the storing part 11 to the brush (coating part) 23.

Also, a coating liquid discharge mechanism 30 is provided in the rear part of the shaft body 10. The above coating liquid discharge mechanism 30 is constituted so that a piston 32 is allowed to move forward in a prescribed distance by rotating a canopy 31 to discharge a prescribed amount of the coating liquid. Also, the brush 23 is covered with a cap 40.

In FIGs. 1 and 2, a code 15 shows a stirring ball for stirring the coating liquid. Also, the coating liquid discharge mechanism 30 described above is disclosed in JP-A 2012-157611 previously applied by the present applicant. The coating liquid discharge mechanism 30 described above may be not only a rotation type in which the coating liquid is discharged by rotating the canopy 31 but also a knock type in which the coating liquid is discharged by pushing the canopy 31.

In the liquid cosmetic applicator A constituted in the manner described above, the stopper 14 is first detached, and the cap 40 (front shaft body 21) is pushed (moved) toward the rear side of the shaft body 10. This allows to bring the end part of the pipe joint 22 into contact with the seal ball 13, and the seal ball 13 is detached from the stopper body 12 to cause the coating liquid to stay in a state in which it can be supplied.

Then, the cap 40 is detached, the canopy 31 is rotated to allow the piston 32 to move forward in a prescribed distance by rotating a canopy 31, whereby a prescribed amount of the coating liquid is discharged to the brush 23 and applied on the applied part of the user.

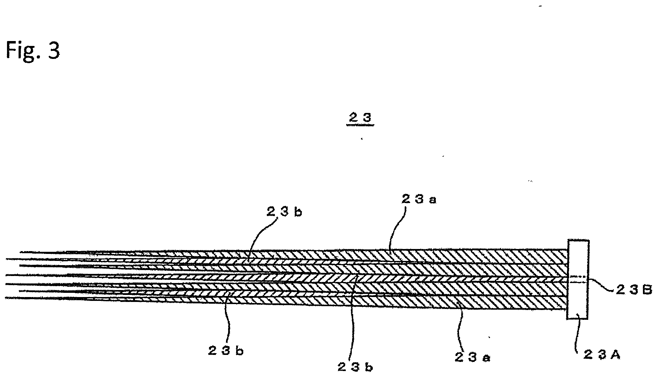

Next, the brush is specifically explained based on FIG. 3 to FIG. 8.

The brush 23 comprises straight synthetic fibers subjected to taper processing at a tip, and it comprises only the fibers having a non-circular deformed cross-sectional shape. In the present embodiment, it is an aggregate of brush hairs 23a and 23b comprising only the fibers having a non-circular deformed cross-sectional shape, and the brush hairs 23a and 23b having a deformed cross-sectional shape are constituted from plural kinds of brush hairs having different cross-sectional shapes.

The above brush hairs 23a and 23b are fused each other at one end thereof so that one annular flange-shaped fused end part 23A is formed.

Also, a hole 23B for improving a distributing property and a supplying property of the coating liquid is provided in the center of the annular flange-shaped fused end part 23A. The hole 23B does not have to be necessarily formed when a liquid such as the coating liquid and the like does not have to be distributed and supplied so frequently.

The brush hairs having a deformed cross-sectional shape mean those in which the shape of the face of the brush hairs perpendicular to a shaft direction is other than a circular shape, and the shape includes, for example, roughly a star shape, roughly a cocoon shape, roughly a cross shape, roughly a triangle shape, roughly a Y shape, roughly a semicircle shape, roughly a rectangle shape, and the like. It includes a shape having at least one or more projections.

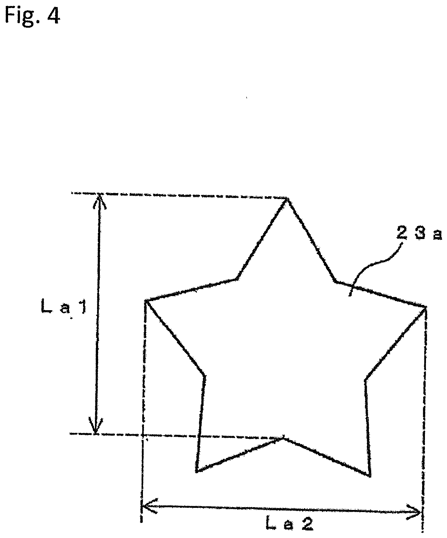

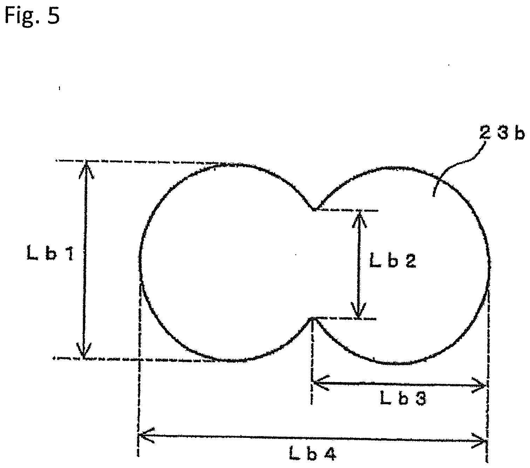

In this respect, the brush hairs having a deformed cross section shall be explained based on FIG. 4 and FIG. 5 with reference to the brush hairs having the cross-sectional shapes of roughly a star shape and roughly a cocoon shape, but the present invention is not limited to roughly the star shape and roughly the cocoon shape, and it is not limited to the brush hairs having two kinds of the deformed cross-sectional shapes. The brush hairs may have at least one kind or two or more kinds of the deformed cross-sectional shapes.

Synthetic resin-made fibers used for the material of the brush hairs include, for example, synthetic resin-made fibers obtained by melting and spinning thermoplastic polymers including polyesters such as polyethylene terephthalate, polybutylene terephthalate, polyethylene terephthalate·isophthalate copolymers and the like, polyolefins such as polyethylene, polypropylene and the like, polyamides such as nylon 6, nylon 610, nylon 612 and the like.

As shown in FIG. 4, a dimension La1 between a concave part and a tip of a convex part opposite thereto in the brush hairs 23a having a cross section of roughly a star shape is in a range of preferably 120 to 200 um, and a dimension La2 between a convex part and a tip of a convex part opposite thereto is in a range of preferably 140 to 250 µm.

Decreasing the dimension La1 described above and increasing the dimension La2 make it possible to deepen (enlarge) a concavity and a convexity which are formed on an outer peripheral surface of the brush hairs and hold a large amount of the coating liquid, and therefore it is preferred. On the other hand, decreasing the dimension La1 described above and increasing the dimension La2 weaken an elasticity of the brush to make it impossible to apply well the coating liquid, and therefore the dimensions are controlled preferably to the ranges described above.

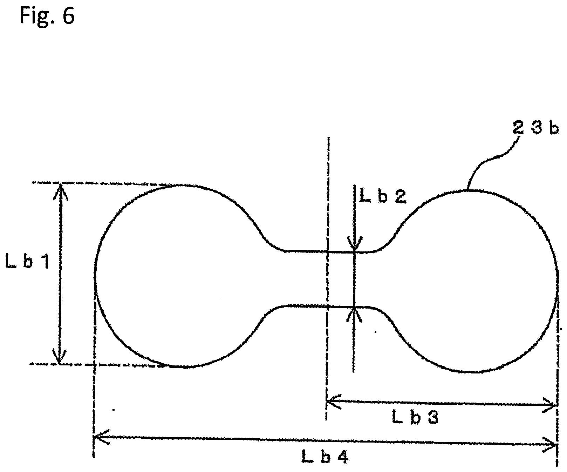

Also, as shown in FIG. 5 and FIG. 6, the brush hairs 23b having a cross section of roughly a cocoon shape have a cross-sectional shape obtained by combining two circles having almost the same shape in the case of FIG. 5, and the brush hairs 23b have a cross-sectional shape obtained by connecting two circles having almost the same shape with a rectangle in the case of FIG. 6.

Also, a depth dimension (a difference between Lb1 and Lb2) of the concave part formed on an outer peripheral surface of the brush hairs 23b having a cross section of roughly a cocoon shape is preferably increased since an amount of the coating liquid contained therein can be increased.

However, when a length dimension Lb2 of a connecting part is too small, a connecting state of two circles is likely to be broken. When broken, the brush hairs are provided with an ordinary circular cross section to make it impossible to increase an amount of the coating liquid contained, and therefore it is not preferred.

Accordingly, controlling the dimensions of Lb1 and Lb3 (dimension to a line symmetric axis) to the ranges of 70 to 200 µm, Lb2 to 20 to 190 µm and Lb4 to 80 to 390 pm, and more preferably Lb1 and Lb3 to the ranges of 100 to 150 µm, Lb2 to 50 to 100 µm and Lb4 to 150 to 300 µm make the brush hairs hard to be divided into two branches when the tips of the brush are subjected to taper processing, and cavities are liable to remain in the taper processing areas, and therefore it is preferred.

Also, the brush 23 is constituted by plural kinds of brush hairs having different cross-sectional shapes, for example, brush hairs having a cross section of roughly a star shape and brush hairs having a cross section of roughly a cocoon shape.

In this respect, use of plural kinds of the brush hairs having different cross-sectional shapes is the preferred embodiment because of the following reasons. (1) When the brush hairs having the same cross-sectional shape are used, concave and convex parts which are formed on the outer peripheral surface of the brush hairs are fit to each other to fill a part or the whole of the concave parts formed on the outer peripheral surface and decrease a gap (aperture) between the brush hairs, and the coating liquid held in the brush is reduced. On the other hand, when plural kinds of the brush hairs having different cross-sectional shapes are used, the concave and convex parts which are formed on the outer peripheral surface of the brush hairs are hard to be fit to inhibit a gap (aperture) between the brush hairs from being reduced. (2) When the brush hairs having the same cross-sectional shape are used to enlarge the concave and convex parts which are formed on the outer peripheral surface of the brush hairs, the brush tends to be less elastic. On the other hand, use of plural kinds of the brush hairs having different cross-sectional shapes makes it possible to provide the brush with a strong elasticity by the other brush hairs even when the concave and convex parts formed on the outer peripheral surface of the brush hairs are enlarged, and the suitable coating property can be obtained.

Also, the sizes of the fibers used for the brush hairs described above shall not be restricted, and used are short fibers having a size of 0.05 to 0.3 mm in terms of a minimum size (diameter) of corresponding diameters of the cross sections vertical to the axis line of the hairs described above, and a length of 5 to 100 mm.

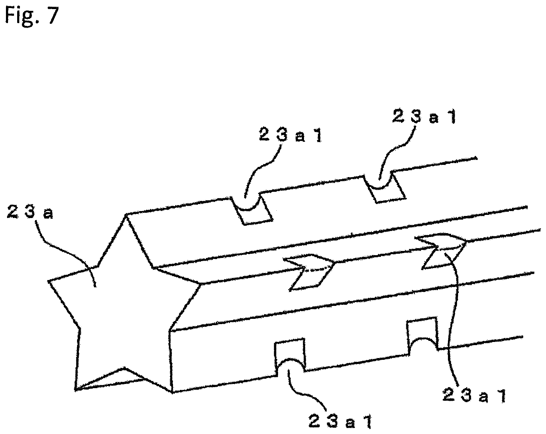

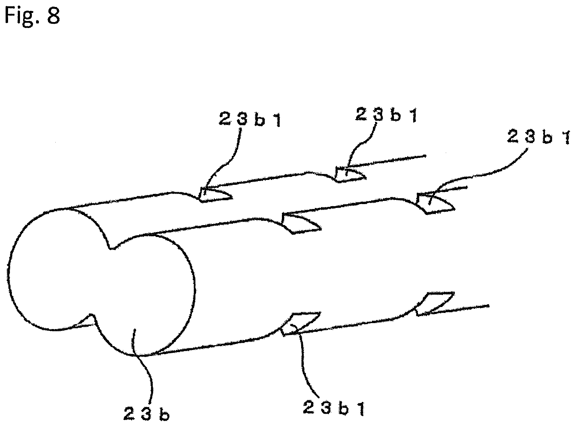

Also, as shown in FIG. 7 and FIG. 8, concave parts 23a1, 23b1 may be formed on the surface parts of the brush hairs (synthetic resin-made fibers) 23a, 23b. As described above, the concave parts 23a1, 23b1 are formed on the fiber surfaces in a longitudinal direction (axis line direction) from the tip parts of the brush hairs 23a, 23b comprising the synthetic resin-made fibers, and therefore many fine gaps are produced between the brush hairs (synthetic resin-made fibers) 23a, 23b.

As a result thereof, the coating liquid is penetrated into the above gaps, and a lot of the liquid is held between the brush hairs (synthetic resin-made fibers) 23a, 23b.

Also, the sizes of the concave parts formed on the surfaces of the fibers shall not specifically be restricted. In general, a depth of the concave part falls in a range of preferably 20 um or less, particularly 5 to 10 pm, and an average pitch of the concave and convex parts falls in a range of preferably 0.1 to 0.5 mm from the viewpoint of the holding force of the liquid.

Also when the concave parts are formed on the surface parts of the brush hairs (synthetic resin-made fibers) 23a, 23b, the corresponding diameter of the cross section vertical to the axis line in the concave part described above is controlled to 0.05 mm or more.

The concave parts 23a1, 23b1 may be formed continuously from one end part side of the brush hairs (synthetic resin-made fibers) 23a, 23b to the other end part side thereof in a spiral form.

Also, a convex part may be formed on the surface parts of the brush hairs (synthetic resin-made fibers) 23a, 23b, and this convex part may be formed continuously from one end part side to the other end part side in a spiral form as is the case with the concave part described above. In general, a height of the convex part falls in a range of preferably 20 um or less, particularly 5 to 10 um, and an average pitch of the convex parts falls in a range of preferably 0.1 to 0.5 mm.

There may be introduced a constitution in which titanium oxide and the like are added to the brush hairs (synthetic resin-made fibers) 23a, 23b to form convex parts on the surface parts thereof to thereby provide gaps between the fibers and in which the fibers are inhibited from being adhered.

Also, the brush 23 is allowed to be constituted by plural kinds of brush hairs having different cross-sectional shapes, and it shall not be restricted to the brush hairs having a cross section of roughly a star shape and the brush hairs having a cross section of roughly a cocoon shape. The cross-sectional shape may be, for example, roughly a cross shape, roughly a triangle shape, roughly a Y shape, roughly a semicircle shape, roughly a rectangle shape, and the like. It is not restricted to two kinds, and the brush hairs having two or more kinds of deformed cross sections may be mixed.

The mixing ratio thereof is suitably set according to the uses of the brush, particularly the degree of the holding property required, and when, for example, the brush hairs having a cross section of roughly a star shape are mixed with the brush hairs having a cross section of roughly a cocoon shape, the mixing ratio thereof is preferably 80 : 20 to 20 : 80 from the viewpoint of substantially enhancing the effect of the holding force.

Also, in the embodiment described above, the brush 23 in which all fibers have a cross section other than a circular shape is used, but the mixture of fibers having a circular cross section and fibers having the respective deformed cross sections described above (the mixing ratio is preferably 50 % or more), and fibers having one (single) deformed cross-sectional shape may be used.

In the liquid cosmetic applicator A constituted in the manner described above, a gap (space) is inhibited from being reduced to make it possible to provide the brush with a strong elasticity when plural kinds of the brush hairs having deformed cross sections which are different in a cross-sectional shape are used for the brush which is the coating part. In addition thereto, the liquid cosmetic composition of the present invention has the characteristics that it does not become blurred on the skin when applied thereon and drops (drips) less and that it has a low viscosity and can draw dense lines, and therefore the synergistic effect of the characteristics of the above liquid cosmetic composition with the structural characteristics of the liquid cosmetic applicator makes it possible to obtain the liquid cosmetic applicator which can charge the brush sufficiently with the liquid cosmetic composition as the coating liquid and can apply the liquid well.

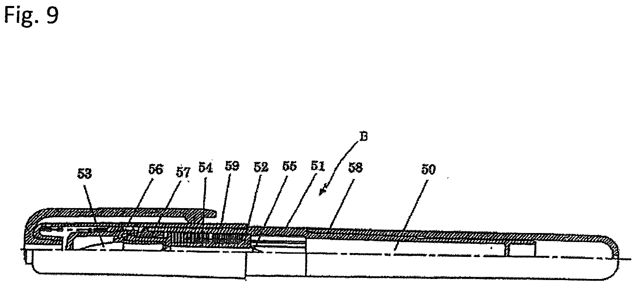

A free ink type liquid cosmetic applicator of a collector type in which the liquid cosmetic composition of the present invention is stored is shown in FIG. 9.

In the above liquid cosmetic applicator B, the liquid cosmetic composition 50 of the present invention is charged in a tank part 51 which is a shaft for directly storing it without allowing it to be stored in a sliver and the like. The applicator B has a constitution in which a single sheet body (ink reservoir, collector member) 52 for storing temporarily the liquid cosmetic composition 50 which is pushed out from the tank part 51 when the air in the tank part 51 is expanded by a rise in the temperature so as to prevent the liquid cosmetic composition 50 from dropping from a pen tip or an air hole is built in the front part of the tank part 51 and in which a pen tip (brush) 53 of a pen type as a coating body is provided in the tip part of the collector member 52. The brush 23 having a deformed cross section used in the liquid cosmetic applicator A described above can be used as the pen tip (brush) 53 of a pen type.

The liquid cosmetic is delivered from the tank part 51 to the pen tip 53 by delivering the liquid cosmetic composition 50 from the tank part 51 to the pen tip 53 which is the coating part via a feed 55 in which a coating liquid passage 54 provided in the central hole of the collector member 52 is provided.

Numbers 56, 57 in FIG. 9 show holder members; a number 58 shows a rear shaft body fixed to a rear part of the tank part 51; and a number 59 shows a cap having an inner cap. Also, the rear part of the pen tip 53 may be arranged directly in the tank part 51 to deliver the liquid cosmetic without passing through the feed 55.

In the liquid cosmetic applicator B of the above embodiment, the cap 59 is detached to thereby allow the applicator to be ready for use as is the case with the liquid cosmetic applicator A.

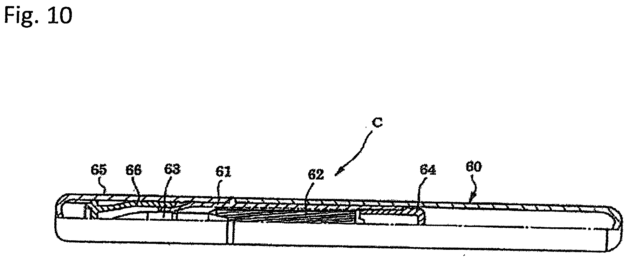

It is shown in FIG. 10 that the liquid cosmetic composition of the present invention is used for a sliver type liquid cosmetic applicator.

In the liquid cosmetic applicator C of the above embodiment, as shown in FIG. 10, an inner shaft 61 is provided in an applicator main body 60; an impregnation body 62 comprising a sliver and the like impregnated with the liquid cosmetic composition of the present invention is received in the inner shaft 61; a coating body 63 comprising brush hairs having a deformed cross section for applying the liquid cosmetic used in the liquid cosmetic applicator A is provided at a tip side of the impregnation body 62; and a tail plug 64 is fixed at a rear end of the inner shaft 61. A number 65 shows a cap body having an inner cap part 66. In the liquid cosmetic applicator C of the above embodiment, the cap 65 is detached to thereby allow the applicator to be ready for use.

In the liquid cosmetic applicator shown in FIG. 9 and FIG. 10 constituted in the manner described above, capable of being obtained is a liquid cosmetic applicator in which the brush can sufficiently be charged with the liquid cosmetic composition of the present invention to make it possible to apply the liquid cosmetic composition in a comfortable way.

In the liquid cosmetic applicators A to C described above, the brushes comprising the brush hairs having a deformed cross section other than a circular shape were used, and since the liquid cosmetic composition of the present invention has, as described above, the characteristics that it does not get blurred on the skin when applied thereon and drops (drips) less and that it has a low viscosity and can draw lines densely, the brushes in the liquid cosmetic applicators A to C may comprise straight synthetic fibers which are subjected to taper processing at tips and which have circular cross sections.

Next, the present invention shall be explained in further details with reference to examples and comparative examples, but the present invention shall not be restricted to the following examples and the like.

### First invention: Examples 1 to 5 and Comparative Examples 1 and 2:

Liquid cosmetic compositions (blend unit: % by mass, whole amount: 100 % by mass) having blend prescriptions shown in the following Table 1 were prepared by wet crushing treatment by means of a media mill and mixing stirring by means of a disper mixer to measure a viscosity value at each shear rate, a particle diameter and a surface tension of each liquid cosmetic composition by the measuring methods described above, and a dripping resistance (liquid holding power), an upper and lower difference of a density (upper and lower difference of a hue) in the brush, a stability A, a coating performance A (presence of blurring, presence of density difference) and a makeup durability were evaluated according to the following respective evaluation methods by means of a liquid cosmetic applicator having the following constitution. The results thereof are shown in the following Table 1.

### Second invention: Examples 6 to 11 and Comparative Examples 3 and 4:

Liquid cosmetic compositions (blend unit: % by mass, whole amount: 100 % by mass) having blend prescriptions shown in the following Table 2 were prepared by wet crushing treatment by means of the media mill and mixing stirring by means of the disper mixer to measure a viscosity value at each shear rate, a particle diameter and a surface tension of each liquid cosmetic composition by the measuring methods described above, and a dripping resistance (liquid holding power), a color unevenness of a brightness (upper and lower difference of a hue) in the brush, a coating property B, a coating performance B (presence of blurring, presence of an unevenness of a brightness) and a makeup durability were evaluated according to the following respective evaluation methods by means of a liquid cosmetic applicator having the following constitution. The results thereof are shown in the following Table 2.

### Constitution of liquid cosmetic applicator:

A liquid cosmetic applicator shown in FIG. 1 to FIG. 3 was used. A brush having the following constitution was used.
(1) Fiber having a star shape cross section (refer to FIG. 4): La1 130 mm, La2 150 mm
   Fiber having a cocoon shape cross section (refer to FIG. 5): Lb1 95 mm, Lb2 75 mm, Lb3 90 mm, Lb4 185 mm, mixing ratio of star : cocoon set to 70 : 30

### Evaluation method of dripping resistance (liquid holding power):

The respective liquid cosmetic compositions (coating liquids) prepared in Examples 1 to 11 and Comparative Examples 1 and 4 were put on a deep dish vessel, and a part of 1.5 mm from the tip of the coating part was dipped in the coating liquid. Then, an amount of the liquid contained in the coating part in a state in which the whole coating part was dipped in the coating liquid was measured to evaluate the dripping resistance according to the following evaluation criteria.

### Evaluation criteria:

Ⓞ: 30 mg or more.
○: 25 mg or more to less than 30 mg.
△: 15 mg or more to less than 25 mg.
×: less than 15 mg.

### Evaluation method of upper and lower difference of a density (upper and lower difference of a hue) in the brush:

The respective liquid cosmetic compositions prepared in Examples 1 to 11 and Comparative Examples 1 and 4 were charged in the applicator to discharge the coating liquid in a brush which was a coating part. Then, the applicator was capped and left standing still for 7 days with the coating part turned downward to functionally evaluate a difference in a density at the tip of the brush and in the vicinity of the front shaft and a difference in a density and a hue of the drawn lines [in Table 2, a color unevenness of a brightness (an upper and lower difference of a hue) in the brush] with naked eyes according to the following evaluation criteria.

### Evaluation criteria:

⊚: difference in a density and a hue was not observed at all in either the brush or the drawn lines.
○: a little difference in a density and a hue was observed in the brush, but when drawing lines, a difference in a density and a hue was not observed at all in the beginning of and after drawing lines.
△: a clear difference in a density and a hue was observed in the brush, and when drawing lines, a clear difference in a density and a hue was observed in the beginning of and after drawing lines.
×: pigment was observed to be settled down in the brush, and lines could not be drawn or even if could not be drawn, blurring was noticeable.

### Evaluation method of stability A:

The respective liquid cosmetic compositions which were the respective internal liquids prepared in Examples 1 to 5 and Comparative Examples 1 and 2 were put in a glass vessel with a lid and stored in a thermostatic bath of 50°C for 10 days to evaluate the appearance thereof according to the following evaluation criteria.

### Evaluation criteria:

○: upper and lower difference in separation were not observed at all.
△: a little supernatant was observed.
×: precipitates were observed, and evenness was not achieved soon by stirring.

### Evaluation method of stability B:

The respective liquid cosmetic compositions which were the internal liquids prepared in Examples 6 to 11 and Comparative Examples 3 and 4 were put in a glass vessel with a lid and stored in a thermostatic bath of 50°C for 7 days to evaluate the appearance thereof according to the following evaluation criteria.

### Evaluation criteria:

○: supernatant of 5 (mm) or less was found.
△: supernatant of exceeding 5 (mm) and 8 (mm) or less was found.
×: supernatant of exceeding 8 (mm) was found.

### Evaluation method of coating performance A (presence of blurring, presence of density difference):

A liquid cosmetic applicator of a brush pen type shown in FIG. 2 was charged with the respective liquid cosmetic compositions prepared in Examples 1 to 5 and Comparative Examples 1 and 2 and used to draw five lines having a width of 1 to 2 mm and a length of about 5 cm on the back of the hand, and the feeling of drawing and a density of the drawn lines were functionally evaluated according to the following evaluation criteria.

### Evaluation criteria:

⊚: blurring and a density difference were not observed, and the lines were drawn densely and easy to draw.
○: a little inferior to the criterion of Ⓞ, and the drawn lines did not become blurred, did not cause a density difference, were easy to draw and satisfactory in a density.
△: the drawn lines caused a little blurring and feathering but were judged to fall in a practical area.
×: the drawn lines caused blurring and feathering and brought about unsatisfactory feeling.

### Evaluation method of coating performance B (presence of blurring, presence of an unevenness of a brightness):

The liquid cosmetic applicator of a brush pen type shown in FIG. 2 was charged with the respective liquid cosmetic compositions prepared in Examples 6 to 11 and Comparative Examples 3 and 4 and used to draw five lines having a width of 1 to 2 mm and a length of about 5 cm on the back of the hand, and the feeling of drawing and a density of the drawn lines were functionally evaluated according to the following evaluation criteria.

### Evaluation criteria:

Ⓞ: blurring and an unevenness of a brightness were not observed, and the lines were drawn densely with a brightness and easy to draw.
○: a little inferior to the criterion of Ⓞ, and the drawn lines did not become blurred, were not uneven in a brightness and easy to draw and had a density with a bright feeling.
△: the drawn lines caused a little blurring and feathering but were judged to fall in a practical area.
×: the drawn lines caused blurring and feathering and brought about unsatisfactory feeling.

### Evaluation method of a makeup durability:

The liquid cosmetic applicator of a brush pen type shown in FIG. 2 was charged with the respective liquid cosmetic compositions prepared in Examples 1 to 11 and Comparative Examples 1 to 4 and used to draw eye lines, and the eye lines after 5 hours passed were observed to functionally evaluate a makeup durability thereof by the state of the eye lines according to the following evaluation criteria.

### Evaluation criteria:

○: no change.
△: a little pale but almost no change.
×: the lines were peeled off in crumbs and expanded, and the makeup was broken.

**Table 1**

| (whole amount: 100 % by mass) | | | | | | | | |
|---|---|---|---|---|---|---|---|---|
| **Category of Used Components** | **Specific Compound Name** | **Examples** | | | | | **Comparative Examples** | |
| | | **1** | **2** | **3** | **4** | **5** | **1** | **2** |
| Inorganic Pigment 1 | Carbon Black * 1 | 8 | 1 | | 1 | 8 | 8 | 1 |
| Inorganic Pigment 2 | Red Iron Oxide * 2 | | 10 | | 10 | | | 10 |
| Inorganic Pigment 3 | Konjo (Prussian blue) * 3 | | | 8 | | | | |
| Aqueous Emulsion 1 | Alkyl Acryliate Copolymer Emulsion #4 | 20 | | 30 | | 30 | 20 | |
| Aqueous Emulsion 2 | Alkyl Acryliate Copolymer Emulsion *5 | | 30 | | 30 | | | |
| Aqueous Emulsion 3 | Alkyl Acrylate Copolymer Emulsion *6 | | | | | | | 30 |
| Crystalline Cellulose | Crystalline Cellulose *7 | 2 | 2 | 2 | 2 | 6 | | 2 |
| Dispersant | Sodium Polyaspartate Aqueous Solution (Concentration 30%) | | 3 | | | | | 3 |
| Surfactant | | 2 | 0.2 | 0.5 | 0.2 | 2 | 2 | 0.2 |
| | Polyethylene Glycol Alkylether Sulfosuccinic Acid Diethlhexyl Na | 0.1 | | | | | 0.1 | |
| | Ethylhexylglycerine | 0.2 | 0.2 | 0.2 | 0.2 | 0.2 | 0.2 | I 0.2 |
| Chelating Agent | EDTA 2Na (Disodium dihydrogen ethylenediamine tetraacetate) | 0.3 | 0.3 | 0.3 | 0.3 | | 0.3 | 0.3 |
| Moisturizing Agent | 1,3-Buthyleneglycol | 10 | 10 | 10 | 10 | 10 | 10 | 10 |
| Preservative | Methylparaben | 0.3 | 0.3 | 0.3 | 0.3 | 0.3 | 0.3 | 0.3 |
| Preservative | Sodium dehydroacetic acid | 0.3 | 0.3 | 0.3 | 0.3 | 0.3 | 0.3 | 0.3 |
| Neutralizing Agent | Aminomethylpropanol | 0.1 | 0.2 | 0.1 | 0.2 | 0.1 | 0.1 | 0.1 |
| | Citric Acid | | 0.1 | | 0.1 | | | |
| Water | Refined Water | Balance | Balance | Balance | Balance | | | |
| Viscosity (mPa· s) | Shear Rate 3.83(S⁻¹) | 100 | 120 | 110 | 100 | 500 | 6 | 115 |
| | Shear Rate 38.3(S⁻¹) | 30 | 40 | 35 | 30 | 120 | 5 5 | 40 40 |
| | Shear Rate 383(S⁻¹) | 10 | 15 | 12 | 10 | 28 | 4 | **14** |
| Surface tension (mN/m) | | 35 | 40 | 35 | 35 | 35 | 35 | 35 |
| Particle Diameter (*µ* m) | | 0.13 | 0.12 | 0.12 | 0.25 | 0.12 | 0.13 | 0.12 |
| Dripping Resistance (Liquid Holding Power) | | ○ | ○ | ○ | ○ | ○ | × | ○ |
| Upper and Lower Difference of a Density in the Brush (Upper and Lower Difference of a Hue) | | ○ | ○ | ○ | Δ | ○ | ○ | ○ |
| Stability A | | ○ | ○ | ○ | Δ | ○ | ○ | ○ |
| Coating Performance A (Presence of Blurring, Presence of Density Difference | | ○ | ○ | ○ | ○ | Δ | × | ○ |
| Makeup Durability | | ○ | ○ | ○ | ○ | ○ | ○ | × |

**Table 2**

| (whole amount 100 % by mass) | | | | | | | | | |
|---|---|---|---|---|---|---|---|---|---|
| **Category of Used Components** | **Specific Compound Name** | **Examples** | | | | | | **Comparative Examples** | |
| | | **8** | **7** | **8** | **9** | **10** | **11** | **3** | **4** |
| Color materials 1 | Red Iron Oxide Coated Titanium Mica *8 | 15 | | 12 | 15 | | 31 | 15 | |
| Color materials 2 | Black Titanium Coated Glass Powder *9 | | 10 | | | 10 | | | 15 |
| Color materials 3 | Black Iron Oxide *10 | | | 3 | | | | | |
| Aqueous Emulsion 1 | Emulsion *4 Alkyl Acrylate Copolymer Emulsion *4 | 15 | | 20 | 15 | | 20 | 20 | |
| Aqueous Emulsion 2 | Alkyl Acrylate Copolymer Emulsion * 5 | | 20 | | | 20 | | | |
| Fermented Cellulose | Fermented Cellulose | 0.2 | 0.2 | 0.2 | 0.05 | 0.6 | 0.2 | | 0.2 |
| Dispersant | Alkyl Acrylate Copolymer *11 | 3 | 3 | 3 | 3 | | | 3 | 3 |
| Surfactant | Polyoxyethylene Alkyl ether | | | | | 3 | 3 | | |
| | Ethylhexylglycerine | 0.2 | 0.2 | 0.2 | 0.2 | 0.2 | 0.2 | 0.2 | 0.2 |
| Chelating Agent | EDTA 2Na (Disodium dihydrogen ethylenediamine tetraacetate) | 0.2 | 0.2 | 0.2 | 0.2 | 0.2 | 0.2 | 0.2 | 0.2 |
| Moisturizing Agent | 1,3-Buthyleneglycol | 10 | 10 | 10 | 10 | 10 | 10 | 10 | 10 |
| Preservative | Methylparaben | 0.3 | 0.3 | 0.3 | 0.3 | 0.3 | 0.3 | 0.3 | 0.3 |
| Preservative | Sodium dehydroacetic acid | 0.3 | 0.3 | 0.3 | 0.3 | 0.3 | 0.3 | 0.3 | 0.3 |
| Water | Refined Water | Balance | Balance | Balance | Balance | Balance | Balance | Balance | Balance |
| Viscosity (mPa· s) | Shear Rate 3.83(S⁻¹) | 97 | 108 | 95 | 65 | 334 | 117 | 34 | 112 |
| | Shear Rate 76.6(S⁻¹) | 65 | 70 | 62 | 44 | 193 | 78 | 25 | 72 |
| | Shear Rate 383(S⁻¹) | 30 | 31 | 28 | 23 | 61 | 38 | 13 | 32 |
| Surface tension (mN/m) | | 40 | 40 | 40 | 40 | 35 | 35 | 40 | 38 |
| Dripping Resistance (Liquid Holding Power) | | ○ | ○ | ○ | Δ | ○ | ○ | × | ○ |
| Upper and Lower Difference of a Density in the Brush (Upper and Lower Difference of a Hue) | | ⊚ | ⊚ | ⊚ | ○ | ⊚ | ⊚ | Δ | Ⓞ |
| Stability B | | ○ | ○ | ○ | Δ | ○ | ○ | × | Δ |
| Coating Performance A (Presence of Blurring, Presence of DensityDifference). | | Ⓞ | ⊚ | ⊚ | ○ | Δ | Δ | × | ○ |
| Makeup Durability | | ○ | ○ | ○ | ○ | Δ | Δ | ○ | × |

The terms *1 to *11 in Table 1 and Table 2 show the followings.
*1: UNIPURE BLACK LC902 (manufactured by Sensient Technologies Corporation)
*2: Red Iron Oxide 216P (manufactured by Daito Kasei Kogyo Co., Ltd.)
*3: Konjo No. 671 (manufactured by Daito Kasei Kogyo Co., Ltd.)
*4: Yodosol GH800F (glass transition point: -15°C, solid content: 45 %, manufactured by Akzo Nobel Corporation)
*5: Yodosol GH810F (glass transition point: 10°C, solid content: 46 %, manufactured by Akzo Nobel Corporation)
*6: Vinysol 1076 (glass transition point: 50°C, solid content: 35 %, manufactured by Daido Chemical Corporation)
*7: CEOLUS RC-N30 (manufactured by Asahi Kasei Chemicals Corporation)
*8: Croizone Golden Bronze, average particle diameter: 30 um, manufactured by BASG AG
*9: METASHINE MT1080RS, average particle diameter: 80 µm, manufactured by Nippon Sheet Glass Co., Ltd.
*10: TAROX BL100P, average particle diameter: 0.4 µm, manufactured by Titan Kogyo, Ltd.
*11: Luvimer 100P, manufactured by BASG AG

As apparent from the results summarized in Table 1 described above, it has been found that the liquid cosmetic compositions prepared in Examples 1 to 5 falling in the scope of the present first invention are excellent in a dripping resistance (liquid holding power), an upper and lower difference of a density (upper and lower difference of a hue) in the brush, a coating performance (presence of blurring, presence of density difference), a stability and a makeup durability as compared with those prepared in Comparative Examples 1 and 2 falling outside the scope of the present first invention.

Observing individually the comparative examples, Comparative Example 1 is a case in which the crystalline cellulose is not contained, and Comparative Example 2 is a case in which the aqueous emulsion of the acrylic acid base polymer has Tg of higher than 20°C, and it has been found that the effects of the present invention cannot be exerted in the above cases.

Also, a fiber cross section of the coating part (brush) 23 was set to a deformed cross section other than a circular shape in the examples described above, and it has been found that also in a case in which the coating part (brush) 23 was provided with the fibers having a circular cross section having an outer diameter φ of 0.15 mm and a whole length of 17 mm and a flange-shaped fused end part 23A having an outer diameter φ of 3 mm and in which the tips of the fibers were processed in a taper form, the liquid cosmetic compositions are a little inferior in a dripping resistance (liquid holding power), an upper and lower difference of a density (upper and lower difference of a hue) in the brush and a coating performance (presence of blurring, presence of density difference) as compared with those prepared in Examples 1 to 3, wherein the respective items were ranked sequentially to the criteria of ○, △, △ and △, and the item of a makeup durability was ranked to the criterion of ○ and that they are excellent as compared with those prepared in Comparative Examples 1 and 2.

As apparent from the results summarized in Table 2 described above, it has been found that the liquid cosmetic compositions prepared in Examples 6 to 11 falling in the scope of the present second invention are excellent in a dripping resistance (liquid holding power), a color unevenness of a brightness (upper and lower difference of a hue) in the brush, a coating performance (presence of blurring, presence of an unevenness of a brightness), a stability and a makeup durability as compared with those prepared in Comparative Examples 3 to 4 falling outside the scope of the present second invention.

Observing individually the comparative examples, Comparative Example 3 is a case in which the fermented cellulose is not blended, and Comparative Example 4 is a case in which the aqueous emulsion is not blended and that the effects of the present invention cannot be exerted in the above cases.

Also, the coating part (brush) 23 was provided with mixed fibers having a circular cross section and a deformed cross section in Examples 6 to 11, and it has been found that also in a case in which the coating part (brush) 23 was provided with the fibers having a circular cross section having an outer diameter φ of 0.15 mm and a whole length of 17 mm and a flange-shaped fused end part 23A having an outer diameter φ of 3 mm and in which the tips of the hairs were processed in a taper form, the liquid cosmetic compositions are a little inferior in a dripping resistance (liquid holding power), an upper and lower difference of a density (upper and lower difference of a hue) in the brush and a coating performance (presence of blurring, presence of an unevenness of a brightness) as compared with those prepared in Examples 6 to 11, wherein the respective items were ranked sequentially to the criteria of △, ○ and ○, and the item of a stability was ranked to the criterion of ○ and that they are excellent as compared with those prepared in Comparative Examples 3 and 4.

### INDUSTRIAL APPLICABILITY

A liquid cosmetic composition suited to eye makeup for an eyeliner or an eyebrow is obtained.

### EXPLANATION of CODES

1: Applicator
10: Shaft body
11: Storing part (receiving part)
12: Stopper body
13: Seal ball
14: Stopper
20: Front shaft
21: Front shaft body
23: Brush (coating part)
23a: Deformed cross-sectional fiber (star shape)
23a1: Concave part
23b: Deformed cross-sectional fiber (cocoon shape)
23b1: Concave part
23A: Flange-shaped fused end part
23B: Hole

## Claims

1. A liquid cosmetic applicator equipped with at least a coating part, a coating liquid reservoir (11), and a front shaft (20), wherein the coating part comprises a brush (23); the fibers of the brush (23) include those having a cross section other than a circle; the coating liquid reservoir (11) stores a liquid cosmetic composition; the front shaft (20) is equipped with a front shaft body (21), a pipe joint (22) which is installed in the front shaft body (21), and a conduit tube (24) for connecting the pipe joint (22) with the coating part; a stopper (14) is installed between the coating part and the coating liquid reservoir (11) and the stopper (14) is detached in starting use to make it possible to supply the liquid cosmetic composition from the coating liquid reservoir (11) to the coating part and a piston (32) of a coating liquid discharge mechanism (30) in the rear part of the shaft body (10) is allowed to move forward in a prescribed distance by rotating or pushing a canopy (31) to discharge a prescribed amount of the liquid cosmetic composition to the coating part; wherein the liquid cosmetic composition comprises at least an aqueous emulsion of an acrylic acid base polymer having a glass transition point of 20°C or lower obtained by polymerizing monomers selected from an acrylic acid monomer, a methacrylic acid monomer, an acrylic ester monomer and a methacrylic ester monomer, crystalline cellulose, an inorganic pigment selected from the following group A, and water, and wherein the liquid cosmetic composition has a viscosity of 20 to 500 mPa-s at a temperature of 25°C and a shear rate of 3.83 s⁻¹ by means of a cone plate type viscometer (ELD type viscometer) and a surface tension of 25 to 60 mN/m at 25 °C measured by a Wilhelmy method using a CBVP-Z type surface tension meter (plate method), Group A:
carbon blacks, iron oxide particles, chromium oxide, ultramarine blue, Prussian blue, zinc oxide, aluminum oxide, silicon dioxide, titanium oxide, magnesium oxide, chromium hydroxide, calcium carbonate, titanium yellow, red iron oxide.

2. The liquid cosmetic applicator as described in claim 1, wherein the liquid cosmetic composition comprises 2 to 30 % by mass (in terms of a solid content) of the aqueous emulsion of the acrylic acid base polymer, 0.1 to 3.0 % by mass of the crystalline cellulose and 1 to 30 % by mass of the inorganic pigment.

3. The liquid cosmetic applicator as described in claim 1 or 2, wherein an average particle diameter of the inorganic pigments in the liquid cosmetic composition is 20 to 300 nm or less determined by measuring at 25 °C according to a dynamic light scattering method.

4. A liquid cosmetic applicator equipped with at least a coating part, a coating liquid reservoir (11), and a front shaft (20), wherein the coating part comprises a brush (23); the fibers of the brush (23) include those having a cross section other than a circle; the coating liquid reservoir (11) stores a liquid cosmetic composition; the front shaft (20) is equipped with a front shaft body (21), a pipe joint (22) which is installed in the front shaft body (21) and a conduit tube (24) for connecting the pipe joint (22) with the coating part; a stopper (14) is installed between the coating part and the coating liquid reservoir (11) and the stopper (14) is detached in starting use to make it possible to supply the liquid cosmetic composition from the coating liquid reservoir (11) to the coating part and a piston (32) of a coating liquid discharge mechanism (30) in the rear part of the shaft body (10) is allowed to move forward in a prescribed distance by rotating or pushing a canopy (31) to discharge a prescribed amount of the liquid cosmetic composition to the coating part; wherein the liquid cosmetic composition comprises at least an aqueous emulsion of an acrylic acid base polymer having a glass transition point of 20°C or lower obtained by polymerizing monomers selected from an acrylic acid monomer, a methacrylic acid monomer, an acrylic ester monomer and a methacrylic ester monomer, fermented cellulose, a color material containing at least a tabular pigment selected from the following group B, and water, and the liquid cosmetic composition has a viscosity of 30 to 200 mPa.s at a temperature of 25°C and a shear rate of 76.6 s⁻¹ by means of a cone plate type viscometer (ELD type viscometer) and a surface tension of 25 to 60 mN/m at 25 °C measured by a Wilhelmy method using a CBVP-Z type surface tension meter (plate method), Group B:
mica titanium, carmine-coated mica titanium, chromium oxide-coated mica titanium, iron oxide-coated mica titanium, iron oxide -carmine-coated mica titanium, iron oxide·Prussian blue-coated mica titanium, ultramarine blue-coated mica titanium, Prussian blue-coated mica titanium, red iron oxide-coated mica, red iron oxide-coated mica titanium, red iron oxide - carmine-coated mica titanium, red iron oxide-iron oxide-coated mica titanium, red iron oxide-Prussian blue-coated mica titanium, red iron oxide-iron oxide·Prussian blue-coated mica titanium, and lustrous pigments prepared by coating metal or metal oxide on a glass flake or a massive flake used as a base material.

5. The liquid cosmetic applicator as described in claim 4, wherein the liquid cosmetic composition comprises 2 to 20 % by mass (in terms of a solid content) of the aqueous emulsion of the acrylic acid base polymer, 0.05 to 0.6 % by mass of the fermented cellulose and 1 to 30 % by mass of the color material containing at least the tabular pigment.

6. The liquid cosmetic applicator as described in claim 4 or 5, wherein the liquid cosmetic composition comprises a pigment other than the tabular pigment selected from the following group C:
Group C:
carbon blacks, iron oxide particles, titanium oxide, Prussian blue, ultramarine blue, blue No. 1, red iron oxide, chromium oxide, chromium hydroxide, zinc oxide, zirconium oxide, cobalt oxide, scale foils, bismuth oxychloride, blue No. 2, blue No. 404, red No. 201, red No. 202, red No. 220, red No. 102, red No. 104, yellow No. 4, and yellow No. 4 Al lake, and an aluminum-coating polyester film.

7. The liquid cosmetic applicator as described in any one of claims 1 to 6, wherein the liquid cosmetic composition is a liquid cosmetic composition for eye makeup.

8. The liquid cosmetic applicator as described in any one of claims 1 to 7, wherein all the fibers of the brush have cross sections other than a circle.

## Patentansprüche

1. Ein Flüssigkosmetik-Applikator, der mit mindestens einem Auftragsteil, einem Auftragsflüssigkeitsreservoir (11) und einem vorderen Schaft (20) ausgestattet ist, wobei der Auftragsteil einen Pinsel (23) umfasst; die Fasern des Pinsels (23) jene mit einem von einem Kreis verschiedenen Querschnitt umfassen; das Auftragsflüssigkeitsreservoir (11) eine flüssige kosmetische Zusammensetzung aufbewahrt; der vordere Schaft (20) mit einem vorderen Schaftkörper (21), einer Rohrverbindung (22), die in dem vorderen Schaftkörper (21) angebracht ist, und einer Rohrleitung (24) zum Verbinden der Rohrverbindung (22) mit dem Auftragsteil ausgestattet ist; ein Stopper (14) zwischen dem Auftragsteil und dem Auftragsflüssigkeitsreservoir (11) angebracht ist und der Stopper (14) zu Beginn der Verwendung entfernt wird, um es zu ermöglichen, die flüssige kosmetische Zusammensetzung aus dem Auftragsflüssigkeitsreservoir (11) dem Auftragsteil zuzuführen, und es einem Kolben (32) eines Auftragsflüssigkeits-Fördermechanismus (30) im hinteren Teil des Schaftkörpers (10) ermöglicht wird, sich durch Drehen oder Drücken einer Abdeckung (31) eine vorgeschriebene Strecke weit vorwärts zu bewegen, um eine vorgeschriebene Menge der flüssigen kosmetischen Zusammensetzung an den Auftragsteil abzugeben; wobei die flüssige kosmetische Zusammensetzung mindestens eine wässrige Emulsion eines Polymers auf Acrylsäurebasis mit einem Glasübergangspunkt von 20°C oder niedriger, das durch Polymerisieren von Monomeren, ausgewählt aus einem Acrylsäuremonomer einem Methacrylsäuremonomer, einem Acrylsäureestermonomer und einem Methacrylsäureestermonomer erhalten wurde, kristalline Cellulose, ein anorganisches Pigment, ausgewählt aus der folgenden Gruppe A, und Wasser umfasst, und wobei die flüssige kosmetische Zusammensetzung eine Viskosität von 20 bis 500 mPa·s bei einer Temperatur von 25°C und einer Scherrate von 3,83 s⁻¹ mittels eines Viskosimeters vom Kegelplattentyp (Viskosimeter vom ELD-Typ) und eine Oberflächenspannung von 25 bis 60 mN/m bei 25°C, gemessen gemäß einer Wilhelmy-Methode unter Verwendung eines Oberflächenspannungsmessgeräts vom Typ CBVP-Z (Plattenmethode), aufweist, Gruppe A:
Ruße, Eisenoxidpartikel, Chromoxid, Ultramarinblau, Preußischblau, Zinkoxid, Aluminiumoxid, Siliciumdioxid, Titanoxid, Magnesiumoxid, Chromhydroxid, Calciumcarbonat, Titangelb, Eisenoxidrot.

2. Der Flüssigkosmetik-Applikator wie in Anspruch 1 beschrieben, wobei die flüssige kosmetische Zusammensetzung 2 bis 30 Massen-% (ausgedrückt als Feststoffgehalt) der wässrigen Emulsion des Polymers auf Acrylsäurebasis, 0,1 bis 3,0 Massen-% der kristallinen Cellulose und 1 bis 30 Massen-% des anorganischen Pigments umfasst.

3. Der Flüssigkosmetik-Applikator wie in Anspruch 1 oder 2 beschrieben, wobei ein mittlerer Teilchendurchmesser der anorganischen Pigmente in der flüssigen kosmetischen Zusammensetzung 20 bis 300 nm oder weniger beträgt, bestimmt durch Messung bei 25°C gemäß einem dynamischen Lichtstreuungsverfahren.

4. Ein Flüssigkosmetik-Applikator, der mit mindestens einem Auftragsteil, einem Auftragsflüssigkeitsreservoir (11) und einem vorderen Schaft (20) ausgestattet ist, wobei der Auftragsteil einen Pinsel (23) umfasst; die Fasern des Pinsels (23) jene mit einem von einem Kreis verschiedenen Querschnitt umfassen; das Auftragsflüssigkeitsreservoir (11) eine flüssige kosmetische Zusammensetzung aufbewahrt; der vordere Schaft (20) mit einem vorderen Schaftkörper (21), einer Rohrverbindung (22), die in dem vorderen Schaftkörper (21) angebracht ist, und einer Rohrleitung (24) zum Verbinden der Rohrverbindung (22) mit dem Auftragsteil ausgestattet ist; ein Stopper (14) zwischen dem Auftragsteil und dem Auftragsflüssigkeitsreservoir (11) angebracht ist und der Stopper (14) zu Beginn der Verwendung entfernt wird, um es zu ermöglichen, die flüssige kosmetische Zusammensetzung aus dem Auftragsflüssigkeitsreservoir (11) dem Auftragsteil zuzuführen, und es einem Kolben (32) eines Auftragsflüssigkeits-Fördermechanismus (30) im hinteren Teil des Schaftkörpers (10) ermöglicht wird, sich durch Drehen oder Drücken einer Abdeckung (31) eine vorgeschriebene Strecke weit vorwärts zu bewegen, um eine vorgeschriebene Menge der flüssigen kosmetischen Zusammensetzung an den Auftragsteil abzugeben; wobei die flüssige kosmetische Zusammensetzung mindestens eine wässrige Emulsion eines Polymers auf Acrylsäurebasis mit einem Glasübergangspunkt von 20°C oder niedriger, das durch Polymerisieren von Monomeren, ausgewählt aus einem Acrylsäuremonomer, einem Methacrylsäuremonomer, einem Acrylsäureestermonomer und einem Methacrylsäureestermonomer erhalten wurde, fermentierte Cellulose, ein Farbmaterial, das mindestens ein tafelförmiges Pigment enthält, das aus der folgenden Gruppe B ausgewählt ist, und Wasser umfasst, und die flüssige kosmetische Zusammensetzung eine Viskosität von 30 bis 200 mPa·s bei einer Temperatur von 25°C und einer Scherrate von 76,6 s⁻¹ mittels eines Viskosimeters vom Kegelplattentyp (Viskosimeter vom ELD-Typ) und eine Oberflächenspannung von 25 bis 60 mN/m bei 25°C, gemessen gemäß einer Wilhelmy-Methode unter Verwendung eines Oberflächenspannungsmessgeräts vom Typ CBVP-Z (Plattenmethode) aufweist,
Gruppe B:
Glimmertitan, Karmin-beschichtetes Glimmertitan, Chromoxid-beschichtetes Glimmertitan, Eisenoxid-beschichtetes Glimmertitan, Eisenoxid-Karmin-beschichtetes Glimmertitan, Eisenoxid-Preußischblau-beschichtetes Glimmertitan, Ultramarinblaubeschichtetes Glimmertitan, Preußischblau-beschichtetes Glimmertitan, Eisenoxidrotbeschichteter Glimmer, Eisenoxidrot-beschichtetes Glimmertitan, Eisenoxidrot-Karmin-beschichtetes Glimmertitan, Eisenoxidrot-Eisenoxid-beschichtetes Glimmertitan, Eisenoxidrot-Preußischblau-beschichtetes Glimmertitan, Eisenoxidrot-Eisenoxid-Preußischblau-beschichtetes Glimmertitan sowie Glanzpigmente, hergestellt durch Auftragen von Metall oder Metalloxid auf eine Glasflocke oder eine massive Flocke, die als Grundmaterial verwendet werden.

5. Der Flüssigkosmetik-Applikator wie in Anspruch 4 beschrieben, wobei die flüssige kosmetische Zusammensetzung 2 bis 20 Massen-% (ausgedrückt als Feststoffgehalt) der wässrigen Emulsion des Polymers auf Acrylsäurebasis, 0,05 bis 0,6 Massen-% der fermentierten Cellulose und 1 bis 30 Massen-% des Farbmaterials, das mindestens das tafelförmige Pigment enthält, umfasst.

6. Der Flüssigkosmetik-Applikator wie in Anspruch 4 oder 5 beschrieben, wobei die flüssige kosmetische Zusammensetzung ein von dem tafelförmigen Pigment verschiedenes Pigment umfasst, das aus der folgenden Gruppe C ausgewählt ist:
Gruppe C:
Ruße, Eisenoxidpartikel, Titanoxid, Preußischblau, Ultramarinblau, Blau Nr. 1, Eisenoxidrot, Chromoxid, Chromhydroxid, Zinkoxid, Zirkonoxid, Kobaltoxid, Zunderfolien, Wismutoxychlorid, Blau Nr. 2, Blau Nr. 404, Rot Nr. 201, Rot Nr. 202, Rot Nr. 220, Rot Nr. 102, Rot Nr. 104, Gelb Nr. 4 und Gelb Nr. 4 Al-Lake, und ein aluminiumbeschichteter Polyesterfilm.

7. Der Flüssigkosmetik-Applikator wie in einem der Ansprüche 1 bis 6 beschrieben, wobei die flüssige kosmetische Zusammensetzung eine flüssige kosmetische Zusammensetzung für Augen-Make-up ist.

8. Der Flüssigkosmetik-Applikator wie in einem der Ansprüche 1 bis 7 beschrieben, wobei alle Fasern des Pinsels von einem Kreis verschiedene Querschnitte aufweisen.

## Revendications

1. Applicateur de cosmétique liquide équipé d'au moins une partie de revêtement, un réservoir de liquide de revêtement (11), et un arbre avant (20), dans lequel la partie de revêtement comprend un pinceau (23) ; les fibres du pinceau (23) englobent celles ayant une section transversale autre qu'un cercle ; le réservoir de liquide de revêtement (11) stocke une composition cosmétique liquide ; l'arbre avant (20) est équipé d'un corps d'arbre avant (21), d'un raccord de tuyau (22) qui est installé dans le corps d'arbre avant (21), et d'un tube de conduite (24) pour connecter le raccord de tuyau (22) à la partie de revêtement ; un arrêtoir (14) est installé entre la partie de revêtement et le réservoir de liquide de revêtement (11) et l'arrêtoir (14) est détaché quand l'utilisation est démarrée pour rendre possible la délivrance de la composition cosmétique liquide depuis le réservoir de liquide de revêtement (11) à la partie de revêtement et un piston (32) d'un mécanisme de décharge de liquide de revêtement (30) dans la partie arrière du corps d'arbre (10) est laissé à se déplacer vers l'avant sur une distance prescrite par rotation ou poussée d'un carrossage (31) pour décharger une quantité prescrite de la composition cosmétique liquide vers la partie de revêtement ; dans lequel la composition cosmétique liquide comprend au moins une émulsion aqueuse d'un polymère à base d'acide acrylique ayant une température de transition vitreuse de 20 °C ou moins, obtenu par polymérisation de monomères choisis parmi un monomère d'acide acrylique, un monomère d'acide méthacrylique, un monomère d'ester acrylate et un monomère d'ester méthacrylate, de la cellulose cristalline, un pigment inorganique choisi dans le groupe A qui suit, et de l'eau, et dans lequel la composition cosmétique liquide a une viscosité de 20 à 500 mPa·s à une température de 25 °C et à une vitesse de cisaillement de 3,83 s⁻¹ au moyen d'un viscosimètre du type plan-cône (viscosimètre de type ELD) et une tension de surface de 25 à 60 mN/m à 25 °C, mesurée par une méthode de Wilhelmy utilisant un dispositif de mesure de tension de surface de type CBVP-Z (du type à plaque),
Groupe A :
noirs de carbone, particules d'oxyde de fer, oxyde de chrome, bleu outremer, bleu de Prusse, oxyde de zinc, oxyde d'aluminium, dioxyde de silicium, oxyde de titane, oxyde de magnésium, hydroxyde de chrome, carbonate de calcium, jaune de titane, oxyde de fer rouge.

2. Applicateur de cosmétique liquide selon la revendication 1, dans lequel la composition cosmétique liquide comprend 2 à 30 % en masse (en termes de teneur en matières sèches) de l'émulsion aqueuse du polymère à base d'acide acrylique, 0,1 à 3,0 % en masse de la cellulose microcrystalline, et 1 à 30 % en masse du pigment inorganique.

3. Applicateur de cosmétique liquide selon la revendication 1 ou 2, dans lequel le diamètre moyen des particules des pigments inorganiques dans la composition cosmétique liquide est de 20 à 300 nm ou moins, déterminé par mesure à 25 °C conformément à une méthode de diffusion dynamique de la lumière.

4. Applicateur cosmétique liquide équipé d'au moins une partie de revêtement, un réservoir de liquide de revêtement (11), et un arbre avant (20), dans lequel la partie de revêtement comprend un pinceau (23) ; les fibres du pinceau (23) englobent celles ayant une section transversale autre qu'un cercle ; le réservoir de liquide de revêtement (11) stocke une composition cosmétique liquide ; l'arbre avant (20) est équipé d'un corps d'arbre avant (21), d'un raccord de tuyau (22) qui est installé dans le corps d'arbre avant (21), et d'un tube de conduite (24) pour connecter le raccord de tuyau (22) à la partie de revêtement ; un arrêtoir (14) est installé entre la partie de revêtement et le réservoir de liquide de revêtement (11) et l'arrêtoir (14) est détaché quand l'utilisation est démarrée pour rendre possible la délivrance de la composition cosmétique liquide depuis le réservoir de liquide de revêtement (11) à la partie de revêtement et un piston (32) d'un mécanisme de décharge de liquide de revêtement (30) dans la partie arrière du corps d'arbre (10) est laissé à se déplacer vers l'avant sur une distance prescrite par rotation ou poussée d'un carrossage (31) pour décharger une quantité prescrite de la composition cosmétique liquide vers la partie de revêtement ; dans lequel la composition cosmétique liquide comprend au moins une émulsion aqueuse d'un polymère à base d'acide acrylique ayant une température de transition vitreuse de 20 °C ou moins, obtenu par polymérisation de monomères choisis parmi un monomère d'acide acrylique, un monomère d'acide méthacrylique, un monomère d'ester acrylate et un monomère d'ester méthacrylate, de la cellulose fermentée, un matériau coloré contenant au moins un pigment tabulaire choisi dans le groupe B qui suit, et de l'eau, et la composition cosmétique liquide a une viscosité de 30 à 200 mPa· s à une température de 25 °C et à une vitesse de cisaillement de 76,6 s⁻¹ au moyen d'un viscosimètre du type plan-cône (viscosimètre de type ELD) et une tension de surface de 25 à 60 mN/m à 25 °C, mesurée par une méthode de Wilhelmy utilisant un dispositif de mesure de tension de surface de type CBVP-Z (du type à plaque),
Groupe B :
mica titane, mica titane revêtu de carmin, mica titane revêtu d'oxyde de chrome, mica titane revêtu d'oxyde de fer, mica titane revêtu d'oxyde de fer et de carmin, mica titane revêtu d'oxyde de fer et de bleu de Prusse, mica titane revêtu de bleu outremer, mica titane revêtu de bleu de Prusse, mica revêtu d'oxyde de fer rouge, mica titane revêtu d'oxyde de fer rouge, mica titane revêtu d'oxyde de fer rouge et de carmin, mica titane revêtu d'oxyde de fer rouge et d'oxyde de fer, mica titane revêtu d'oxyde de fer rouge et de bleu de Prusse, mica titane revêtu d'oxyde de fer rouge, d'oxyde de fer et de bleu de Prusse, et pigments à reflets lustrés préparés par déposition sous forme de revêtement d'un métal ou d'un oxyde métallique sur une paillette de verre ou une paillette massive utilisée en tant que matériau de base.

5. Applicateur de cosmétique liquide selon la revendication 4, dans lequel la composition cosmétique liquide comprend 2 à 20 % en masse (en termes de teneur en matières sèches) de l'émulsion aqueuse du polymère à base d'acide acrylique, 0,05 à 0,6 % en masse de la cellulose fermentée et 1 à 30 % en masse du matériau coloré contenant au moins le pigment tubulaire.

6. Applicateur de cosmétique liquide selon la revendication 4 ou 5, dans lequel la composition cosmétique liquide comprend un pigment autre que le pigment tabulaire choisi dans le groupe C suivant :
Groupe C :
noirs de carbone, particules d'oxyde de fer, oxyde de titane, bleu de Prusse, bleu outremer, bleu N° 1, oxyde de fer rouge, oxyde de chrome, hydroxyde de chrome, oxyde de zinc, oxyde de zirconium, oxyde de cobalt, feuilles d'écailles, oxychlorure de bismuth, bleu N° 2, bleu N° 404, rouge N° 201, rouge N° 202, rouge N° 220, rouge N° 102, rouge N° 104, jaune N° 4, et jaune N° 4 Al laque, et un film de polyester à revêtement en aluminium.

7. Applicateur de cosmétique liquide selon l'une quelconque des revendications 1 à 6, dans lequel la composition cosmétique liquide est une composition cosmétique liquide pour le maquillage des yeux.

8. Applicateur de cosmétique liquide selon l'une quelconque des revendications 1 à 7, dans lequel toutes les fibres du pinceau ont des sections transversales autres qu'un cercle.
